# EUROPEAN PATENT SPECIFICATION

(11) **EP 2 061 437 B1**
(45) Date of publication and mention of the grant of the patent: **30.10.2013**
(21) Application number: 07793677.1
(22) Date of filing: 24.08.2007
(51) Int. Cl.: A61K 9/22, A61K 9/20, A61K 9/52, A61K 9/16, A61K 31/22, A61K 31/366, A61K 31/404, A61K 9/50, A61K 31/44, A61K 9/28, A61K 9/48

(54) **COMBINED PHARMACEUTICAL FORMULATION WITH CONTROLLED-RELEASE COMPRISING DIHYDROPYRIDINE CALCIUM CHANNEL BLOCKERS AND HMG-COA REDUCTASE INHIBITORS**
KOMBINIERTE PHARMAZEUTISCHE FORMULIERUNG MIT KONTROLLIERTER FREISETZUNG MIT DIHYDROPYRIDIN-CALCIUM-KANALBLOCKERN UND HMG-COA-REDUCTASE-HEMMERN
COMPOSITION PHARMACEUTIQUE COMBINÉE À LIBÉRATION CONTRÔLÉE COMPRENANT DE LA DIHYDROPYRIDINE, DES BLOQUEURS DE CANAL CALCIUM ET DES INHIBITEURS DE L'HGM-COA RÉDUCTASE

(30) Priority: 24.08.2006 KR 20060080694; 24.08.2007 KR 20070085480
(43) Date of publication of application: 27.05.2009
(73) Proprietor: HanAll Biopharma Co., Ltd., Daejeon 306-120 (KR)
(72) Inventor: KIM, Sung Wuk, Gyeonggi-do 463-802 (KR); JUN, Sung Soo, Gyeonggi-do 463-050 (KR); JO, Young Gwan, Daejeon 305-811 (KR); KOO, Ja-Seong, Daejeon 305-756 (KR); KIM, Jin Wook, Daejeon 302-120 (KR)
(74) Representative: Pohlman, Sandra M.
(86) International application number: PCT/KR2007/004079
(87) International publication number: WO 2008/023958

(56) References cited:
- WO-A1-99/11263
- WO-A1-2006/071077
- WO-A2-99/06035
- WO-A2-2004/075892
- WO-A2-2007/103528
- WO-A2-2009/010810
- US-A1- 2003 092 745
- NISHIO S ET AL: "Interaction between amlodipine and simvastatin in patients with hypercholesterolemia and hypertension", HYPERTENSION RESEARCH. CLINICAL AND EXPERIMENTAL, OSAKA, JP, vol. 28, no. 3, 1 March 2005 (2005-03-01), pages 223-227, XP008083125, ISSN: 0916-9636
- NISHIO S. ET AL.: 'Interaction between amlodipine and simvastatin in patients with hypercholesterolemia and hypertension' HYPERTENS. RES. vol. 28, no. 3, March 2005, pages 223 - 227, XP008083125
- MITAL S. ET AL.: 'Simvastatin acts synergistically with ACE inhibitors or amlodipine to decrease oxygen consumption in rat hearts' J. CARDIOVASC. PHARMACOL. vol. 36, no. 2, August 2000, pages 248 - 254, XP002250967

## Description

### [Technical Field]

The present invention relates to a chronotherapeutical combination pharmaceutical formulation with controlled-release comprising a dihydropyridine, calcium channel blocker and a statin, a lipid-lowering agent. The formulation of the present invention is designed in such a manner that the release of each ingredient can be controlled at a predetermined rate by using the so-called chronotherapy, where drugs are administered to exhibit the pharmacological activities at certain time intervals thereby providing improved therapeutic effects while minimizing side effects.

### [Background Art]

Atherosclerosis and hypertension deteriorate symptoms even worse, and these can be prevented only by treatment of both atherosclerosis and hypertension at the same time in patients suffering from hyperlipidemia and hypertension [Hypertens Res 2001; 24: 3-11, Hypertens Res 2003; 26:1-36, Hypertens Res 2003; 26: 979-990].

Thus, there have been clinical results reported about the synergistic effect of the co-administration of a lipid-lowering agent, a statin and a calcium channel blocker as the anti-hypertensive According to Kramsch et al., a combination of amlodipine and a lipid-lowering agent shows a better therapeutic effect for atherosclerosis [Journal of Human Hypertension(1995) (Suppl.1), 53-59]. Jukema et al. also proved the synergistic effect when a calcium channel blocker and a lipid-lowering agent when both are combined [Circulation, 1995 (Suppl.1), 1-197].

A combination of amlodipine as a representative calcium channel blocker, particularly a dihydropyridine, a calcium channel blocker, along with simvastatin as a statin, a lipid-lowering agent, is most widely prescribed.

It is well known that amlodipine serves as a medication for treatment of angina as well as hypertension. Simvastatin is also well known to have a lipid-lowering activity and an anti-inflammatory activity on the wall of blood vessels. The safety of these drugs are also well known and are available in a drug store in Great Britain as over-the-counter(OTC) drugs not requiring prescription [Cardiology 1992; 80 (Suppl 1): S31-S36, J Cardiovasc Pharmacol 1988; 12 (Suppl 7): S110-S113, Lancet 2000; 356:359-365*,* Hypertens Res 2002; 25: 717-725, Hypertens Res 2002; 25: 329-333].

Meanwhile, amlodipine serves as an anti-hypertensive medicine and also increases the lipid-lowering activity of simvastatin through synergistic activity with the lipid-lowering agent. Simvastatin serves as a lipid-lowering agent and also has an activity of decreasing blood pressure through a synergistic effect with amlodipine. The aforementioned two drugs are both administered once daily, and the medication for both drugs is preferred to be administered with dinner.

As a representative statin, a lipid-lowering agent, simvastatin has the following characteristics. That is, it is well known that HMG-CoA reductase inhibitor, a statin, a lipid-lowering agent, is the first option for prevention and treatment of heart diseases due to coronary atherosclerosis, angina or myocardial infarction [Lancet 1995; 346: 750-753, Am J Cardiol 1998; 82: 57T-59T, Am J Cardiol 1995; 76: 107C-112C, Hypertens Res 2003; 26: 699-704, Hypertens Res 2003; 26: 273-280.]Br Med Bull 2001; 59: 3-16, Am J Med 1998; 104 (Suppl 1): 6S-8S, Clin Pharmacokinet 2002; 41: 343-370.].

Moreover, simvastatin is most frequently prescribed among statins, lipid-lowering agents, and has been well known to decrease the rate of coronary atherosclerosis and the death rate through a large-scale clinical test *[*Lancet 1994; 344:1383-1389.].

The aforementioned activities are due to the fact that simvastatin strongly inhibits HMG-CoA reductase, which plays a key role in the synthesis of cholesterol in liver and also inhibits an inflammation-inducing factor ["Scandinavian Simvastatin Survival Study" published in the Lancet, 1994,344, 1383-89].

Patients suffering from atherosclerosis or diabetes show abnormal NO synthase (eNOs) in blood vessel wall, and the blood pressure increases due to the decrease in NO generation. Statin, a lipid-lowering agent including simvastatin, increases the e-NOS to a normal level, which is also an effect of a combined prescription where a lipid-lowering activity helps an anti-hypertensive activity [Am J Physiol Renal Physiol Vol 281 Issue 5: F802-F809, 2001].

Simvastatin is an inactive lactone, which, after entering liver, is transformed into an activated form, simvastatin acid, with lipid-lowering activity. The remaining simvastatin is also metabolized through various steps by cytochrome P450 3A4 in liver, and some of metabolites show a strong lipid-lowering activity.

The simvastatin and simvastatin acid are metabolized by cytochrome P450 3A4, functions in liver and excreted from liver *[*Drug Metab Dispos 1990; 18: 138-145, Drug Metab Dispos 1990; 18: 476-483, Drug Metab Dispos 1997; 25: 1191-1199.].

Thus, when used in combination with a drug inhibiting cytochrome P-450 3A4 enzyme, simvastatin is subject to less metabolism in liver and the plasma concentration of simvastatin is increased, which may lead to serious side effects such as rhabdomyolysis. [Clin Pharmacol Ther 1998; 63: 332-341, Clin Pharmacol Ther 1998; 64: 177-182, Physicians Desk Reference 2006 (Zocor), J Pharmacol Exp Ther 1997; 282: 294-300, Pharmacol Exp Ther 1999; 290:1116-1125, Life Sci 2004; 76: 281-292.].

Therefore, a very specially designed administration should be employed in the co-administration of such drugs like amlodipine that inhibits the induction of the cytochrome P450 3A4 enzyme, together with such drugs like simvastatin that should be metabolized to the active form by the same enzyme cytochrome P450 3A4. Besides, statin-based drugs have been recommended to be administered early in the evening because lipid synthesis in liver becomes very active from the evening. [Arterioscler Thromb 11: 816-826, Clinic Oharmacol Ther 40: 338-343.].

A representative calcium channel blocker, amlodipine, is known as follows.

A calcium channel blocker is the anti-hypertensive medicine that is most frequently prescribed in combination with simvastatin. Particularly, amlodipine is the most widely prescribed in the world as an anti-hypertensive medicine and a medicine for coronary arterial ischemic diseases like angina. [Cardiology 1992; 80 (Suppl 1): S31-S36, J Cardiovasc Pharmacol 1988; 12 (Suppl 7): S110-S113, Lancet 2000; 356: 359-365, Hypertens Res 2002; 25: 717-725, Hypertens Res 2002; 25: 329-333.].

Amlodipine, which is used in the present invention in combination with statin, a lipid-lowering agent represented by simvastatin, is a long-acting drug belonging to dihydropyridine, calcium channel blocker [Clin Pharmacokinet 1992; 22: 22-31, Am Heart J 1989; 118: 1100-1103, Hypertens Res 2003; 26: 201-208.].

Amlodipine, which has a chemical name of 3-ethyl-5-methyl-2-(2-amino ethoxymethyl)-4-(2-chlorophenyl)-1,4-dihydro-6-methyl-3,5-pyridinedicarboxylate, is a very useful calcium channel blocker that has a half-life of 30-50 hours and shows an activity for a relatively long period of time [European patent publication No. 89,167 and U.S. patent No. 4,572,909]. Amlodipine is also a medicine for treating hypertension, which prevents calcium from flowing into vascular smooth muscle and induces the relaxation of peripheral arteries, thereby lowering blood pressure. Also, amlodipine is a useful drug for treating ischemic diseases caused by spasmodic contraction of coronary arterial walls.

When orally administered in the form of a single pill, amlodipine is absorbed in small intestine. Then, more than 40% is metabolized in liver and only the remaining 60% is present in blood, thus sufficiently exerting an activity of lowering blood pressure.

Amlodipine continues the activity for 24 hours, and shows strongest activity of lowering blood pressure during the time from morning to noon when administered in the evening of the previous day.

From a pathophysiological point of view, the high pressure increase in the day time is caused by the spasm of vascular wall due to stress stimulus. Amlodipine functions by causing the relaxing of the spasmodic contraction of vascular wall, and shows a strong activity of lowering blood pressure in the day time. Thus, amlodipine administered during the evening reaches the maximum plasma concentration in the early morning and shows strong activity during the time of morning stress [Hypertens 10(Supp;4) : S136, Clin Invest 72: 864-869].

In the presence of cytochrome P450 3A4 enzyme, some amlodipines are oxidized by the enzyme and metabolized into an inactive material. However, amlodipine immediately shows an activity of inhibiting the generation of cytochrome P450 3A4 enzyme.

Due to the aforementioned nature, amlodipine should be administered at certain time intervals when co-administered with a statin, a lipid-lowering agent such as simvastatin, because amlodipine inhibits the cytochrome P450 3A4 enzyme needed by simvastatin. [Med Chem 1991; 34: 1838-1844, Eur J Clin Pharmacol 2000; 55: 843-852.].

The co-administration of amlodipine and simvastatin has had such delicate problems as mentioned above, but so far it has been very difficult to formulate such combination products free of such problems. It is today's tendency that the prescription of the co-administration of amlodipine and Simvastatin has been continuously practiced by physicians but rarely being found that appropriate instructions for medication would usually be given to patients. Therefore, the majority of patients do not know that simvastatin at least should be taken in the evening and amlodipine should be taken after an interval of a certain number of hours.

However, the co-administration of the aforementioned drugs may increase the plasma concentration of simvastatin acids by about 30% thus generating side effects. It is also difficult to expect the synergistic effects of two drugs in lowering blood pressure and lipid.

Shinichiro Nishio et al, reported the result of experiments comparing two groups of patients suffering from hypertension with hyperlipidemia. One group was administered with amlodipine single pill and simvastatin single pill at the same time, and the other group was administered with simvastatin single pill only [Hypertens Res, 2005, Vol.28, No.3].

According to the experiments, the simultaneous co-administration of simvastatin and amlodipine inhibits cytochrome P450 3A4 enzyme due to amlodipine and increases the plasma concentration of simvastatin by 30% leading to the possibility of side effects.

**Table 1**

| **Dally dosage** | **Cmax (ng/ml)** | **AUC (ng/ml)** |
|---|---|---|
| **Simvastatin 5mg** | **9.6 ± 3.7** | **34.3 ± 16.5** |
| **Simvastatin 5mg + Amlodipine 5mg** | **13.7 ± 4.7** | **43.9 ± 16.6** |
| Hypertension Research Vol.25(2005). No. 3 March 223-227 | | |

As shown in Table 1, as compared to the administration of simvastatin only, the combined prescription was higher by 30% in the plasma concentration of the lipid-lowering ingredient. Nevertheless, the lipid-lowering activity was not increased. At a higher plasma concentration than a certain level, simvastatin decreases in activity of inhibiting biosynthesis of cholesterol, and is likely to incur serious side effects such as rhabdomyolysis.

Korean patent No. 582347 discloses a combination formulation where the amlodipine is dissolved and absorbed at the same time that the statin-based component is simultaneously released for 24 hours. The concept of this patent is completely in the opposite way against the concept of the present invention. However this formulation (Korean patent No. 582347) definitely shows such defect that the two drug components are simultaneously mingled in liver from the first time of the dissolution and an antagonistic drug interaction occurs; that is, amlodipine inhibits the induction of cytochrome P450 3A4 enzyme needed by simvastatin. As a result, some simvastatin acids are pushed out of the liver before being completely metabolized by cytochrome P 450 3A4 into the blood. Increasing the plasma concentration of simvastatin unnecessarily bring the side effects like as rhabdomyolysis.

Korean patent No. 742432 discloses a combination formulation comprising amlodipine camsylate and simvastatin, and a preparation method thereof. In this patent, the two ingredients are released and metabolized in a liver simultaneously. Such formulation is a simple combination where two drug components are simultaneously dissolved, absorbed and entering into the liver. So amlodipine inhibits the induction of cytochrome P450 3A4 to be needed by simvastatin for its activation and complete metabolism in the liver. As a result about 30% of simvastatins are released into blood without acting completely and being metabolized in liver. Such unnecessary higher concentration of simvastatins does not contribute to lowering lipid but increasing side effects. Such defect of simple combination has been ascertained by preliminary clinical test disclosed in the present invention.

Further, Korean patent publication No. 2000-7002144 by Pfizer was also rejected by Korean Intellectual Property Office as being a simple formulation containing amlodipine and atorvastatin.

Thus, there are needs for developing a novel medication method or a novel pharmaceutical formulation that may prevent the shortcoming of the conventional techniques, i.e., such conventional combination products consisting of amlodipine and statin-based drug properties in that these two components are simultaneously absorbed or amlodipine firstly absorbed than simvastain.

### [Disclosure]

### [Technical Problem]

Therefore, there has been a longfelt need for the development of a novel medication method or a novel pharmaceutical formulation that may prevent the drawback of the combination product of two drug components, i.e., the antagonistic effects between the drugs.

As a result, a specially combination pharmaceutical formulation that may overcome the antagonistic effects between drugs was developed in the present invention by taking advantage of the pharmaceutical concept that the drugs may be dissolved at prescheduled intervals.

The present inventors have exerted extensive researches to develop a way to solve the aforementioned problems and increase the therapeutic effect of the combined prescription, which is clinically inevitable, while reducing the side effects.

A clinical test herein proves that the chronotherapeutic administration of a statin, a lipid-lowering agent represented by simvastatin, and a dihydropyridine, a calcium channel blocker represented by amlodipine, has remarkably improved therapeutic effect and safety as compared to that of the simultaneous co-administration of the drugs.

The present invention has found that a time interval between the absorption of a statin, a lipid-lowering agent, and a dihydropyridine, a calcium channel blocker, in the gastrointestinal tract may inhibit any higher plasma concentration of the statin, a lipid-lowering agent than are necessary and prevent various relevant side effects thereof, and developed a combined formulation herein, thereby finally completing the present invention.

In the case of a complex system of the present invention, statin, lipid-lowering agent is absorbed first immediately after the administration of the formulation and transformed into an activated form, by cytochrome P450 3A4 enzyme, and acting in the liver and then further metabolized by cytochrome P450 3A4 to be eliminated through bile duct. After enough time from when simvastatin is affected by cytochrome P450 3A4 enzyme in the liver, amlodipine is absorbed in the gastrointestinal tract. Therefore simvastatin may not be affected by the inhibitory action of amlodipine on the induction of cytochrome P450 3A4.

Considering that simvastatin enters the liver as a first-pass, and is metabolized into active form to inhibit the cholesterol synthesis in liver, the present invention is intended to formulate the combination products in such a way that simvastatin may sufficiently stay in liver for its full lipid lowering activity but not being released into the blood with a higher plasma concentration than a certain level. For this purpose the present invention is characterized in its specially designed controlled releasing formulation.

That is, the present invention is related to control the controlled-release ingredients by constituting the formulation comprising a delayed-release portion containing a dihydropyridine, a calcium channel blocker, and an immediate-release part containing a statin, a lipid-lowering agent, as active ingredients, thus enabling the dihydropyridine, a calcium channel blocker, to be dissolved or absorbed in the small intestine 3-4 hours later than that of the statin.

As compared with the simply formulated combination product of two drug components (calcium channel blocker/statin tablet), the chronotherapeutically combination pharmaceutical formulation of the present invention comprising a dihydropyridine, a calcium channel blocker, and a statin, a lipid-lowering agent, is expected to show far superior clinical effects and safety.

The combination products of the present invention when orally administered, provide a synergistic effect of the two drug components (calcium channel blocker/statin) in the clinical effect and the safety by maximizing the pharmaceutical activity of each active ingredient, by minimizing the antagonistic effects of two drugs in liver and through the control of release of each drug components to maintain its absorption with the lapse of time. For such purpose, the combination product of the present invention is to be administered one tablet daily in the evening preferably between 5 pm and 10 pm.

In principle, a drug should not be administered in combination with another drug if the co-administration results in harm than benefit.

Considering the great synergistic clinical effects of a combined administration of a dihydropyridine, calcium channel blocker and a statin, lipid-lowering agent, particularly amlodipine and simvastatin, the present invention maintains the synergistic effects and also eliminates the side effects by resolving the side effect of simvastatin, e.g., myopathy, which may appear when the two drugs simply being co-administered. Such antagonistic relationship between amlodipine and simvastatin is based on the simple fact that each drug is related to the same cytochrome P 450 3A4 in such antagonistic way that unnecessary simvastatin acids are increased in blood.

Therefore, the present invention aims to provide a functional chronotherapeutic combination pharmaceutical formulation comprising a controlled-release dihydropyridine, calcium channel blocker and a statin, lipid-lowering agent.

### [Technical Solution]

The present invention relates to a combined pharmaceutical formulation comprising a dihydropyridine, a calcium channel blocker, and a statin, a lipid-lowering agent, as active ingredients and a pharmaceutically acceptable carrier, the combined pharmaceutical formulation including a controlled-release part comprising the dihydropyridine, a calcium channel blocker, and an immediate-release part comprising the statin, a lipid-lowering agent, as active ingredients.

### [Advantageous Effects]

As mentioned herein, the present invention applies the chronotherapeutic theory and xenobiotic theory in the controlled release formulation to maximize the pharmacokinetic and pharmacodynamic effects and to minimize the side effects, which may occur when two drugs are simply co-administered.

The formulation of the present invention comprises as active ingredients, statin, a lipid-lowering agent, and dihydropyridine, a calcium channel blocker, and both of ingredients are related to the same cytochrome P450 enzyme, in such a manner that one is affecting on while the other is affected by that enzyme, and is characterized in that the release rates of the aforementioned ingredients are different and the dissolution and absorption of each drug are initiated at certain intervals of time in a controlled manner.

As a result, the formulation of the present invention is more useful pharmacologically, clinically, scientifically and economically in the treatment of a chronic circulatory disorder than when the two drugs are co-administered

Moreover, the combined pharmaceutical formulation of the present invention causes the drugs to be released at different rates, and prevents the antagonistic effects and side effects, while maintaining the synergistic effect of the drugs.

Further, the combined pharmaceutical formulation of the present invention is administered with a single dose once daily in the evening, and has an advantage of convenience in medication and medication instruction.

### [Description of Drawings)

Figure 1 shows a graph comparing dissolution rates between the amlodipine/ simvastatin two-phase matrix tablets prepared in Example 1 and the control drugs (Zocor^{®}: simvastatin single pill, Norvasc^{®}: amlodipine single pill).
Figure 2 shows a graph comparing dissolution rates between the amlodipine/ simvastatin combined pharmaceutical formulation prepared in Examples 4 and 10 and the control drugs (Zocor^{®}: simvastatin single pill, Norvasc^{®}: amlodipine single pill).
Figure 3 shows a graph comparing dissolution rates between the amlodipine/ lovastatin combined pharmaceutical formulation prepared in Example 11 and the control drugs (Mevacor^{®}: lovastatin single pill, Norvasc^{®}: amlodipine single pill).
Figure 4 shows a graph comparing dissolution rates between the amlodipine/atorvastatin combined pharmaceutical formulation prepared in Example 13 and the control drugs (Lipitor^{®}: atorvastatin single pill, Norvasc^{®}: amlodipine single pill).
Figure 5 shows a graph comparing dissolution rates between the lercanidipine/ simvastatin combined pharmaceutical formulation prepared in Example 16 and the control drugs (Zocor^{®}: simvastatin single pill, Zanidip^{®}: lercanidipine single pill).
Figure 6 shows a graph comparing dissolution rates between the lacidipine/ simvastatin combined pharmaceutical formulation prepared in Example 18 and the control drugs (Zocor^{®}: simvastatin single pill, Vaxar : lacidipine single pill).
Figure 7 shows results of clinical test in Experimental Example 7, which compares the plasma concentrations of simvastatin acid between experimental groups.
Figure 8 shows results of clinical test in Experimental Example 7, which compares the plasma concentrations of simvastatin acid between experimental groups.
Figure 9 shows results of clinical test in Experimental Example 7, which compares the plasma concentrations of simvastatin and simvastatin acid between experimental groups.
Figure 10 shows results of clinical test in Experimental Example 7, which compares the plasma concentrations of amlodipine between experimental groups.

### [Best Mode]

Hereunder is provided a detailed description of the present invention.

The present invention relates to a chronotherapeutic combined pharmaceutical formulation, which is designed such that the release of each ingredient may be controlled to a predetermined rate by applying the principle of the so-called chronotherapy and the xenobiotics, where drugs are administered so that the activities of the drugs are effective chronotherapeutically without any antagonistic interaction of drugs. The formulation of the present invention comprises as active ingredients, a statin, a lipid-lowering agent, and a dihydropyridine, a calcium channel blocker, both of which are related to the same cytochrome P450 enzyme, in such a manner that one is affecting on while the other is affected by that enzyme, and is characterized in that the release rates of the aforementioned ingredients are different and the dissolution and absorption of each drug are initiated at certain intervals of time in a controlled manner, thereby preventing antagonistic effects and side effects, while maintaining the synergistic effect and providing convenience in medication.

Hereunder is provided a detailed description of the combined pharmaceutical formulation according to the present invention, which comprises a dihydropyridine, calcium channel blocker and a statin, lipid-lowering agent.

The combined pharmaceutical formulation of the present invention comprises a dihydropyridine, a calcium channel blocker, and a statin, a lipid-lowering agent as active ingredients. Such a dihydropyridine, a calcium channel blocker, is known as a substance inhibiting the induction of cytochrome P450 3A4. Examples of the dihydropyridine, a calcium channel blocker, include amlodipine, lercanidipine, lacidipine and a pharmaceutically acceptable salt thereof. Preferably, amlodipine or a pharmaceutically acceptable salt thereof or an isomer thereof, specifically amlodipine maleate and amlodipine besylate, may be used as the dihydropyridine, a calcium channel blocker. Preferable daily dosage of the dihydropyridine, calcium channel blocker of the present invention is 1-20 mg (for a male adult weight 65-75 kg), preferably 5-10 mg to be contained in a tablet (total weight: 200-500 mg).

As the dihydropyridine, calcium channel blocker having an efficacy of lowering blood pressure, the present application specifically describes amlodipine. However, the present invention shall not be limited to amlodipine.

At least one selected from the group consisting of simvastatin, lovastatin, atorvastatin, pitavastatin, rosuvastatin, fluvastatin, pravastatin and a pharmaceutically acceptable salt thereof may be used as the statin, lipid-lowering agents. The daily dosage of the statin, lipid-lowering agent of the present invention is 5-160 mg, preferably 5-80 mg for an adult to be contained in a tablet (total weight: 200-500 mg).

Representative example of the statin, a lipid-lowering agent, is simvastatin, and the present invention describes simvastatin as a specific example. However, the present invention is in no way limited to simvastatin. Although simvastatin is inactive material, it may be changed into an active simvastatin acid by esterase, and further changed into an activated form by cytochrome P450 3A4 in liver, thereby exerting a lipid-inhibiting activity.

Meanwhile, amlodipine inhibits the induction of enzyme cytochrome P450 3A4. Therefore, when amlodipine and simvastatin are administered at the same time, amlodipine that is rapidly absorbed into the small intestine reaches liver earlier than simvastatin and thereby inhibits the induction of cytochrome P450 3A4. Hence, a considerable portion of the simvastatin that reaches the liver later or at the same time is not subject to the metabolic activity of cytochrome P450 3A4 and about 30% of the simvastatins(simvastatin, simvastatin acids, etc.) may enter the systemic bloodstream before they are fully metabolized by cytochrome P450 3A4 to be active in liver or excreted through the bile duct. As a result, the necessary high concentration of simvastatin or simvastatin acid in the blood may cause muscular disorder like rhabdomyolysis.

As a way to solve the aforementioned problem and prevent amlodipine from inhibiting the full enzymatic metabolic pathway of simvastatin in liver, the present invention constitutes an immediate-release part that releases simvastatin first and causes simvastatin to be absorbed by the small intestine earlier, while constituting a controlled-release part amlodipine to be absorbed into liver 3-4 hours later than that of simvastatin.

The novel composition of the present invention comprises a controlled-release composition containing amlodipine, a pharmaceutically acceptable salt thereof and desired additives and an immediate-release composition containing simvastatin and desired additives, which is physically separated or partitioned so that two different drugs show different release rates. Moreover, the immediate-release part and the controlled-release part may be formulated into various forms.

That is, the novel pharmaceutical composition may be coated according to a conventional method by using a release controlling material selected among the group comprising the controlled-release part, and thus obtained coated particles or granules and multi-component particles or granules of an immediate-release simvastatin composition may be compressed into a tablet or filled in a capsule.

The controlled-release part of the present invention contains a dihydropyridine, a calcium channel blocker such as amlodipine, and an enteric polymer, a water-insoluble polymer, a hydrophobic compound, a hydrophilic nonpolymeric compound and a hydrophilic polymer as a release controlling material thereof. The release controlling material in the controlled-release part may be contained in an amount of 10-500 weight parts relative to 100 weight parts of the dihydropyridine, calcium channel blocker. If the amount is below the above range, the release control may not be sufficient. If the amount is above the range, the release of drug is delayed and statistically significant clinical effect may not obtained.

Examples of the enteric polymer include but are not limited to polyvinylacetate phthalate, methacrylic acid copolymer, hydroxypropylmethyl cellulose phthalate, shellac, cellulose acetate phthalate, cellulose propionate phthalate, Eudragit L, Eudragit S and a mixture thereof may be used.

Examples of the water-insoluble polymer include but are not limited to a pharmaceutically acceptable polyvinylacetate, methacrylic acid copolymer such as poly(ethylacrylate-co-methylmethacrylate) copolymer or poly(ethylacrylate-methyl methacrylate-trimethyl aminoethyl methacrylate) copolymer, ethyl cellulose, cellulose acetate and a mixture thereof may be used.

Examples of the hydrophobic organic compound include but are not limited to a fatty acid and a fatty acid ester, a fatty acid alcohol, a wax, an inorganic material and a mixture thereof may be used. Specifically, examples of the fatty acid and fatty acid esters include but are not limited to glyceryl palmitostearate, glyceryl stearate, glyceryl behenate, cetyl palmitate, glyceryl mono oleate, stearic acid and a mixture thereof may be used; examples of the fatty acid alcohol include but are not limited to cetostearyl alcohol, cetyl alcohol, stearyl alcohol and a mixture thereof; examples of the wax include but are not limited to Carnauba wax, beeswax, microcrystalline wax and a mixture thereof; and the examples of the inorganic material include but are not limited to talc, precipitated calcium carbonate, dibasic calcium phosphate, zinc oxide, titanium oxide, kaolin, bentonite, montmorillonite, veegum and a mixture thereof.

Examples of the hydrophilic polymer include but are not limited to a saccharide, a cellulose derivative, a gum, a protein, a polyvinyl derivative, a polymethacrylate copolymer, a polyethylene derivative, a carboxyvinyl polymer and a mixture thereof. Specifically, examples of the saccharide include but are not limited to dextrin, polydextrin, dextran, pectin and pectin derivative, alginate, poly(galacturonic acid), xylan, arabinoxylan, arabinogalactan, starch, hydroxypropyl starch, amylase, amylopectin and a mixture thereof; examples of the cellulose derivative include but are not limited to hydroxypropylmethyl cellulose, hydroxypropyl cellulose, hydroxymethyl cellulose, hydroxyethyl cellulose, methyl cellulose, carboxymethyl cellulose sodium, hydroxypropyl methyl cellulose acetate succinate, hydroxyethylmethyl cellulose and a mixture thereof; examples of the gums include but are not limited to guar gum, locust bean gum, tragacantha, carrageenan, gum acasia, gum arabic, gellan gum, xanthan gum and a mixture thereof; examples of the proteins include but are not limited to gelatin, casein, zein and a mixture thereof; examples of the polyvinyl derivative include but are not limited to polyvinyl alcohol, polyvinyl pyrrolidone, polyvinylacetal diethylaminoacetate and a mixture thereof; examples of the polymethacrylate copolymer include but are not limited to poly(butyl methacrylate, (2-dimethylaminoethyl)methacrylate, methylmethacrylate) copolymer, poly(methacrylic acid, methylmethacrylate) copolymer, poly(methacrylic acid, ethylacrylate) copolymer and a mixture thereof; examples of the polyethylene derivative include but are not limited to polyethylene glycol, polyethylene oxide and a mixture thereof; and examples of the carboxyvinylpolymer include but are not limited to carbomer.

The formulation of the present invention may further comprise such amounts of other additives that the effect of the present invention may not be damaged. Examples of a pharmaceutically acceptable diluent as the aforementioned additives include without limitation starch, microcrystalline cellulose, lactose, glucose, mannitol, alginate, salt of alkaline earth metal, clay, polyethylene glycol and dicalcium phosphate. Examples of a lubricant as the aforementioned additives include without limitation talc, magnesium stearate and alkaline earth metal stearate type calcium, zinc, etc., lauryl sulfate, hydrogenated vegetable oil, sodium benzoate, sodium stearyl fumarate, glyceryl monostearate and polyethylene glycol 4000.

The controlled-release part of the present invention consists of discontinuous phases of particles or granules prepared by mixing, granulating or coating a dihydropyridine, as a calcium channel blocker, a release controlling material and commonly used pharmaceutical additives.

The immediate-release part of the present invention may be prepared into particles or granules by performing normal processes for manufacture of oral solid forms such as mixing, combining, drying and granulation using statin, lipid-lowering agent such as simvastatin as an active ingredient and a pharmaceutically acceptable additive. If the flowability of simvastatin mixture is good enough for direct compression, the mixture may be mixed to provide composition, while if the fluidity is not good, a composition may be prepared by compaction, granulation and grinding, thus enabling to prepare a continuous phase comprising an immediate-release part.

A formulation for oral administration comprising a controlled-release part and an immediate-release part matrix in two phases by post-mixing a composition contained in the controlled-release part and the immediate-release part with pharmaceutically acceptable additives for compression or by filling the composition into a capsule.

For example, the formulation according to the present invention may be prepared into two-phase matrix partitioned in a single pill by granule phase, multi-layered tablet, inner core tablet or a capsule filled with granules of a controlled-release part and an immediate-release part. Moreover, the formulation may also be prepared into a tablet comprising a controlled-release inner core tablet containing amlodipine and an immediate-release double inner core tablet containing simvastatin.

However, the formulation according to the present invention is not limited to a single two-phase matrix tablet where a discontinuous phase of a controlled-release amlodipine exists in a continuous phase of an immediate-release simvastatin.

That is, a tablet for oral administration having layers for an immediate-release or a controlled-release by mixing granules contained in the controlled-release part and the immediate-release part with pharmaceutically acceptable additives, followed by compression into a double-layered or a triple-layered tablet where layers are parallel to each other using a compressor for the production of a multi-layered tablet.

Moreover, a tablet for oral administration having a structure of a controlled-release layer as an inner core and an immediate-release layer encompassing the inner core by mixing and compressing the granulates contained in the controlled-release part with pharmaceutically acceptable additive to provide an inner core tablet and by mixing and compressing the granulates contained in the immediate-release part with a pharmaceutically acceptable additive.

Further, a capsule formulation for oral administration, where the two-phase release-control is possible, can be obtained by mixing granulates contained in the controlled-release part and the immediate-release part with a pharmaceutically acceptable additive and filling the mixture in a capsule.

Examples of the pharmaceutical additives that constitute the controlled-release part of the formulation herein include a diluent, a binder, a disintegrant, a lubricant, a stabilizer, a colorant and a flavor. Preferable amount of these additives is 100-3,000 weight parts with regard to 100 weight parts of the statin, lipid-lowering agent. Besides the active ingredient and the release controlling material, the formulation of the present invention may further comprise such amounts of other additional ingredients that the effect of the present invention may not be damaged. Examples of a pharmaceutically acceptable diluent as the aforementioned additives include without limitation starch, microcrystalline cellulose, lactose, glucose, mannitol, alginate, a salt of alkaline earth metal, clay, polyethylene glycol and dicalcium phosphate. Examples of binder as the aforementioned additives include without limitation starch, microcrystalline cellulose, highly-dispersed silica, mannitol, lactose, polyethylene glycol, polyvinylpyrrolidone, hydroxypropyl methyl cellulose, hydroxypropyl cellulose, natural gum, synthetic gum, copovidone and gelatin. Examples of a disintegrant as the aforementioned additives include without limitation starch or denatured starch such as sodium starch glycolate, corn starch, potato starch and pre-gelatinated starch; clay such as bentonite, montmorillonite and veegum; celluloses such as microcrystalline cellulose, hydroxypropyl cellulose and carboxymethyl cellulose; aligns such as sodium alginate or alginic acid; crosslinked celluloses such as croscarmellose sodium; gums such as guar gum and xanthan gum; a crosslinked polymer such as crospovidone; and effervescent formulation such as sodium bicarbonate and citric acid. Examples of a lubricant as the aforementioned additives include without limitation talc, magnesium stearate and alkaline earth metal stearate type calcium, zinc, etc., lauryl sulfate, hydrogenated vegetable oil, sodium benzoate, sodium stearyl fumarate, glyceryl monostearate and polyethylene glycol 4000. Other pharmaceutically acceptable additives such as coloring agents or perfumery may be used.

Although microcrystalline cellulose, sodium starch glycolate, colloidal silicon dioxide, magnesium stearate, etc are used in Examples herein as the additives, the present invention is in no way limited to the aforementioned additives, and the usage of the additives may be easily determined by one skilled in the art.

The formulation may optionally comprise a coating layer on the surface of the tablet. That is, the amlodipine/simvastatin combined pharmaceutical formulation of the present invention may be formulated into an uncoated form or a coated tablet for better stability of active ingredients.

The coating layer may be formed on the surface of tablet using the aforementioned ingredients by conventional methods such as a fluidized-bed coating method and, preferably, a pan coating method.

The coating layer may be prepared by using a film former, a film-forming adjuvant or a mixture thereof. In particular, the coating layer may be prepared by using cellulose derivative such as hydroxypropylmethyl cellulose and hydroxypropyl cellulose, saccharide derivative, polyvinyl derivative, waxes, lipids, gelatin and a mixture as a film former; polyethylene glycol, ethyl cellulose, glycerides, titanium oxide, diethyl phthalate and a mixture thereof as a film-forming adjuvant.

The coating layer is preferred to be contained in an amount of 0.5-15 wt% of total weight of the coated tablet.

The combined pharmaceutical formulation of the present invention is prepared into a single combined pharmaceutical formulation containing amlodipine and simvastatin as active ingredients, and may be administered once daily in the evening.

Hence, comparing with the case of the co-administration of two drugs simultaneously or the case of the administration of each drugs separately with time intervals, the combined pharmaceutical formulation of the present invention has advantages of easy medication instructions, providing the benefit to minimize the side effects and reduced efficacy to be caused by the antagonistic effects between the drugs.

When administered orally, the combined pharmaceutical formulation of the present invention shows an immediate-release of simvastatin and releases more than 80% of initial amount of simvastatin within one hour. The release of amlodipine is sufficiently controlled in a small intestine, which begins 2 hours after the administration, and the amount of release until 3 hours after the administration does not exceed 40% of initial amount of amlodipine. It is preferable that simvastatin releases more than 90% of initial amount within one hour, and that the released amount of amlodipine does not exceed 30% of initial amount until 3 hours after the administration.

Further, the present invention discloses results of a clinical test, which compares the therapeutic effects of (i) a single administration of commercially available statin, lipid-lowering agent (simvastatin 20 mg), (ii) a simultaneous co-administration of the commercially available statin, lipid-lowering agent and a commercially available dihydropyridine, calcium channel blocker (amlodipine besylate 5 mg) and, as an experimental group, (iii) a chronotherapeutic administration of the commercially available statin, lipid-lowering agent and the dihydropyridine, calcium channel blocker. The chronotherapeutic administration was so designed that the release rate of the drugs are the same as in a combined formulation in the present invention.

As a result, it was ascertained that the chronotherapeutic group shows a remarkable improvement in therapeutic efficacy and safety than the simultaneous co-administration group, and that such therapeutic efficacy and safety seemed to be partially due to the changes in the plasma concentration of drugs and partially due to the full synergistic effects of two drugs.

The present inventors have exerted extensive researches to find effective methods to formulate the combination product of the present invention.

The combined pharmaceutical formulation of the present invention may be used for prevention and treatment of hypertension, atherosclerosis, hyperlipidemia, cerebro-cardiovascular ischemic diseases, stroke, renal ischemic disease, etc.

### [Mode for Invention]

The present invention is described more specifically by the following Examples. Examples herein are meant only to illustrate the present invention, but they should not be construed as limiting the scope of the present invention.

### Example 1: Preparation of amlodipine-simvastatin inner core tablets

### 1) Preparation of amlodipine controlled-release layer

Predetermined amounts of amlodipine and microcrystalline cellulose as shown in Table 2 were sieved with a No. 35 sieve, and mixed using a double cone mixer. The mixture was placed into a fluidized-bed granulator (GPCG 1: Glatt), and sprayed with a binder solution (an aqueous solution of hydroxypropylmethyl cellulose) to prepare granules, and dried. The granules were combined with carbomer 71G powders, and mixed with magnesium stearate with a double cone mixer. The resulting mixture was compressed using a rotary compressor (MRC-33: Sejong) at a speed of 30 revolutions pe r minute(rpm) to provide tablets with a hardness of 7-9 kp, a thickness of 3.0 mm and a diameter of 5.5 mm.

### 2) Preparation of a simvastatin layer

Predetermined amounts of simvastatin, microcrystalline cellulose and mannitol as shown in Table 2 were sieved with a No. 35 sieve and mixed using a high-speed mixer. A binder solution prepared by dissolving hydroxypropyl cellulose and citric acid in water, and combined with the mixture containing the main ingredients. Thus obtained mixture was granulated using an oscillator with a No. 20 sieve, dried at 60 °C using a steam dryer, ground with a No. 20 sieve. The resulting mixture was mixed with butylatedhydroxyanisole, sodium starch glycolate and colloidal silicon dioxide, and finally mixed with magnesium stearate using a double cone mixer.

### 3) Compression and coating

The composition was compressed using a compressor for the production of a multi-layered tablet (MRC-37: Sejong). In detail, the composition comprising simvastatin was input in a first power inlet and the composition comprising amlodipine was input in a second inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

### Example 2: Preparation of amlodipine-simvastatin two-phase matrix tablets

### 1) Preparation of amlodipine controlled-release granules

Predetermined amounts of amlodipine and microcrystalline cellulose as shown in Table 2 were sieved with a No. 35 sieve and mixed. The mixture was mixed using Kollicoat SR30D in a high-speed mixer. Thus obtained mixture was granulated using oscillator with a No. 20 sieve, dried at 60 °C using a steam dryer and sized with a No. 20 sieve.

### 2) Preparation of simvastatin granules

Predetermined amounts of simvastatin, microcrystalline cellulose and mannitol as shown in Table 2 were sieved with a No. 35 sieve and mixed using a high-speed mixer. A binder solution was prepared by dissolving hydroxypropyl cellulose and citric acid in water and combining with the mixture of the main ingredients. Thus obtained mixture was combined, granulated using an oscillator with a No. 20 sieve, dried at 60 °C using a steam dryer, ground with a No. 20 sieve, and mixed with butylatedhydroxyanisole.

### 3) Post-mixing, compression and coating

The obtained composition was mixed using a double cone mixer, added with sodium starch glycolate and colloidal silicon dioxide, and finally mixed with magnesium stearate.

The final composition was compressed using a rotary compressor (MRC-33: Sejong) at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing two-phase matrix tablets.

### Example 3: Preparation of amlodipine-simvastatin two-phase matrix tablets

### 1) Preparation of amlodipine controlled-release granules

Predetermined amounts of amlodipine and microcrystalline cellulose as shown in Table 2 were sieved with a No. 35 sieve, and mixed using a double cone mixer. The mixture was placed into a fluidized-bed granulator (GPCG 1: Glatt), and sprayed with a binder solution (an aqueous solution of hydroxypropylmethyl cellulose) to prepare granules. After the granules were dried, they were coated by spraying a 5 wt% solution of Eudragit RS PO in a 1:1 mixture of ethanol and methylene chloride.

### 2) Preparation of simvastatin granules

Predetermined amounts of simvastatin, microcrystalline cellulose and mannitol as shown in Table 2 were sieved with a No. 35 sieve and mixed using a high-speed mixer. A binder solution was prepared by dissolving hydroxypropyl cellulose and citric acid in water and combining with the mixture of the main ingredients. Thus obtained mixture was granulated using an oscillator with a No. 20 sieve, dried at 60 °C using a steam dryer, ground with a No. 20 sieve, and mixed with butylatedhydroxyanisole.

### 3) Post-mixing, compression and coating

The obtained composition was mixed using a double cone mixer, added with sodium starch glycolate and colloidal silicon dioxide, and mixed with magnesium stearate using a high-speed mixer.

The final composition was compressed using a rotary compressor (MRC-33: Sejong) at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing two-phase matrix tablets.

### Example 4: Preparation of amlodipine-simvastatin multi-layered tablets

### 1) Preparation of amlodipine controlled-release layer

Predetermined amounts of amlodipine and microcrystalline cellulose as shown in Table 2 were sieved with a No. 35 sieve, and mixed using a double cone mixer. The mixture was sprayed with a binder solution (an aqueous solution of hydroxypropylmethyl cellulose) to prepare granules. After the granules were dried, they were coated by spraying a 5 wt% solution of hydroxypropylmethyl cellulose phthalate in a 1:1 mixture of ethanol and methylene chloride. The coated granules were mixed with magnesium stearate using a double cone mixer.

### 2) Preparation of simvastatin layer

Predetermined amounts of simvastatin, microcrystalline cellulose and mannitol as shown in Table 2 were sieved with a No. 35 sieve and mixed using a high-speed mixer. A binder solution was prepared by dissolving hydroxypropyl cellulose and citric acid in water and combining with the mixture of the main ingredients. Thus obtained mixture was combined, granulated using an oscillator with a No. 20 sieve, dried at 60 °C using a steam dryer and ground with a No. 20 sieve. The granules were mixed with butylatedhydroxyanisole, sodium starch glycolate and colloidal silicon dioxide, and finally mixed with magnesium stearate using a double cone mixer.

### 3) Compression and coating

The composition was compressed using a compressor for the production of a multi-layered tablet (MRC-37: Sejong). In detail, the composition comprising simvastatin was input in a first power inlet and the composition comprising amlodipine was input in a second inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

### Example 5: Preparation of amlodipine-simvastatin two-phase matrix tablets

### 1) Preparation of amlodipine controlled-release granules

Predetermined amounts of amlodipine and microcrystalline cellulose as shown in Table 2 were sieved with a No. 35 sieve, and mixed using a double cone mixer. The mixture was placed into a fluidized-bed granulator (GPCG 1: Glatt), and sprayed with a binder solution (an aqueous solution of hydroxypropylmethyl cellulose) to prepare granules. After drying, the granules were coated by spraying a 5 wt% solution of hydroxypropylmethyl cellulose phthalate in a 1:1 mixture of ethanol and methylene chloride.

### 2) Preparation of simvastatin granules

Predetermined amounts of simvastatin, microcrystalline cellulose and mannitol as shown in Table 2 were sieved with a No. 35 sieve and mixed using a high-speed mixer. A binder solution was prepared by dissolving hydroxypropyl cellulose and citric acid in water, and combined with the mixture of the main ingredients. The combined mixture was granulated using an oscillator with a No. 20 sieve, dried at 60 °C using a steam dryer, ground with a No. 20 sieve, and mixed with butylatedhydroxyanisole.

### 3) Post-mixing, compression and coating

The obtained composition was mixed using a double cone mixer, added with sodium starch glycolate and colloidal silicon dioxide, and mixed with magnesium stearate using a high-speed mixer.

The final composition was compressed using a rotary compressor (MRC-33: Sejong) at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing two-phase matrix tablets.

### Example 6: Preparation of amlodipine-simvastatin multi-layered tablets

### 1) Preparation of amlodipine controlled-release layer

Predetermined amounts of amlodipine and microcrystalline cellulose as shown in Table 2 were sieved with a No. 35 sieve, and mixed using a double cone mixer. The mixture was sprayed with a binder solution (an aqueous solution of hydroxypropylmethyl cellulose) to prepare granules. After the granules were dried, they were coated by spraying a 5 wt% solution of Eudragit RS PO in a 1:1 mixture of ethanol and methylene chloride. The coated granules were mixed with magnesium stearate using a double cone mixer.

### 2) Preparation of simvastatin layer

Predetermined amounts of simvastatin, microcrystalline cellulose and mannitol as shown in Table 2 were sieved with a No. 35 sieve and mixed using a high-speed mixer. A binder solution was prepared by dissolving hydroxypropyl cellulose and citric acid in water, and combining with the mixture of the main ingredients in a high-speed mixer. Thus obtained mixture was granulated using an oscillator with a No. 20 sieve, dried at 60 °C using a steam dryer and ground with a No. 20 sieve. The granules were mixed with butylatedhydroxyanisole, sodium starch glycolate and colloidal silicon dioxide, and finally mixed with magnesium stearate using a double cone mixer.

### 3) Compression and coating

The composition was compressed using a compressor for the production of a multi-layered tablet (MRC-37: Sejong). In detail, the composition comprising simvastatin was input in a first power inlet and the composition comprising amlodipine was input in a second inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

### Example 7: Preparation of amlodipine-simvastatin inner core tablets

### 1) Preparation of amlodipine core tablet

Predetermined amounts of amlodipine and microcrystalline cellulose as shown in Table 2 were sieved with a No. 35 sieve, and mixed using a double cone mixer. The mixture was introduced into a fluidized-bed granulator (GPCG 1: Glatt) and sprayed with a binder solution (an aqueous solution of hydroxypropylmethyl cellulose) to prepare granules. After the granules were dried, they were coated by spraying a 5 wt% solution of hydroxypropylmethyl cellulose phthalate in a 1:1 mixture of ethanol and methylene chloride. The coated granules were mixed with magnesium stearate using a double cone mixer and compressed using a rotary compressor (MRC-33; Sejong) at a rate of 30 revolutions per minute(rpm) to provide tablets with a hardness of 7-9 kp, thickness of 3.0 mm and a diameter of 5.5 mm, which was used as core tablets.

### 2) Preparation of simvastatin layer

Predetermined amounts of simvastatin, microcrystalline cellulose and mannitol as shown in Table 2 were sieved with a No. 35 sieve and mixed using a high-speed mixer. A binder solution was prepared by dissolving hydroxypropyl cellulose and citric acid in water, and combining with the mixture of the main ingredients in a high-speed mixer. Thus obtained mixture was granulated using an oscillator with a No. 20 sieve, dried at 60 °C using a steam dryer and ground with a No. 20 sieve. The granules were mixed with butylatedhydroxyanisole using a double cone mixer.

### 3) Compression and coating

Compression was performed with a compressor for the production of an inner core tablet (KUD-1: Kilian) at a rate of 30 revolutions per minute(rpm) using the amlodipine core tablet and the composition comprising simvastatin as an inner core and an outer layer, respectively, to provide a tablet with a hardness of 7-9 kp, a thickness of 6.0 mm and a diameter of 9.5 mm. Hi-coater (SFC-30N, Sejong mechanics, Korea), thus producing inner core tablets.

### Example 8: Preparation of amlodipine-simvastatin inner core tablets

### 1) Preparation of amlodipine core tablets

Predetermined amounts of amlodipine and microcrystalline cellulose as shown in Table 2 were sieved with a No. 35 sieve, and mixed using a double cone mixer. The mixture was introduced into a high-speed mixer, combined with Kollicoat SR30D and granulated using an oscillator with a No. 20 sieve. After the granules were dried, they were ground with a No. 20 sieve. The sized granules were mixed with magnesium stearate using a double cone mixer and compressed using a rotary compressor (MRC-33; Sejong) at a rate of 30 revolutions per minute(rpm) to provide tablets with a hardness of 7-9 kp, thickness of 3.0 mm and a diameter of 5.5 mm, which was used as core tablets.

### 2) Preparation of simvastatin layer

Predetermined amounts of simvastatin, microcrystalline cellulose and mannitol as shown in Table 2 were sieved with a No. 35 sieve and mixed using a high-speed mixer. A binder solution was prepared by dissolving hydroxypropyl cellulose and citric acid in water, and combining with the mixture of the main ingredients in a high-speed mixer. Thus obtained mixture was granulated using an oscillator with a No. 20 sieve, dried at 60 °C using a steam dryer and ground with a No. 20 sieve. The granules were mixed with butylatedhydroxyanisole using a double cone mixer.

### 3) Compression and coating

Compression was performed with a compressor for the production of an inner core tablet (KUD-1: Kilian) at a rate of 30 revolutions per minute(rpm) using the amlodipine core tablet and the composition comprising simvastatin as an inner core and an outer layer, respectively, to provide a tablet with a hardness of 7-9 kp, a thickness of 6.0 mm and a diameter of 9.5 mm. Hi-coater (SFC-30N, Sejong mechanics, Korea), thus producing inner core tablets.

### Example 9: Preparation of amlodipine-simvastatin two-phase capsules

### 1) Preparation of amlodipine controlled-release granules

Predetermined amounts of amlodipine and microcrystalline cellulose as shown in Table 2 are sieved using a No. 35 sieve, mixed using a double cone mixer. The mixture was introduced into a fluidized-bed granulator (GPCG 1: Glatt), granulated by spraying a binder solution (an aqueous solution of hydroxypropylmethyl cellulose) and dried. The granules were coated by spraying a 5 wt% solution prepared by dissolving hydroxypropylmethyl cellulose phthalate in a 1:1 mixture of ethanol and methylene chloride.

### 2) Preparation of simvastatin granules

Predetermined amounts of simvastatin, microcrystalline cellulose and mannitol as shown in Table 2 were sieved with a No. 35 sieve and mixed and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60 °C using a steam dryer and ground with a No. 20 sieve. The sized granules was added with butylatedhydroxyanisole and finally mixed using a double cone mixer.

### 3) Compression and coating

The resulting composition prepared in the aforementioned processes 1) and 2) was mixed using a double cone mixer and added with sodium starch glycolate. The mixture was mixed using a double cone mixer, further mixed with colloidal silicon dioxide and finally mixed with magnesium stearate. The resulting mixture was introduced into a powder inlet and filled using a capsule filling machine.

### Example 10: Preparation of amlodipine-simvastatin two-phase capsules

### 1) Preparation of amlodipine controlled-release granules

Predetermined amounts of amlodipine and microcrystalline cellulose as shown in Table 2 were sieved using a No. 35 sieve and mixed using a double cone mixer. The mixture was introduced into a fluidized-bed granulator (GPCG 1: Glatt), granulated by spraying Kollicoat SR30D and dried.

### 2) Preparation of simvastatin granules

Predetermined amounts of simvastatin, microcrystalline cellulose and mannitol as shown in Table 2 were sieved using a No. 35 sieve and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60 °C using a steam dryer and ground with a No. 20 sieve. The sized granules were finally mixed with butylatedhydroxyanisole.

### 3) Compression and coating

The resulting composition prepared in the aforementioned processes 1) and 2) was mixed using a double cone mixer and added with sodium starch glycolate. The mixture was mixed using a double cone mixer, further mixed with colloidal silicon dioxide using a double cone mixer and finally mixed with magnesium stearate. The resulting mixture was introduced into a powder inlet and filled using a capsule filling machine.

### Example 11: Preparation of amlodipine-lovastatin multi-layered tablets

### 1) Preparation of amlodipine controlled-release layer

Predetermined amounts of amlodipine and microcrystalline cellulose as shown in Table 3 were sieved with a No. 35 sieve and mixed using a double cone mixer. The mixture was introduced into a fluidized-bed granulator (GPCG 1: Glatt), granulated by spraying a binder solution (an aqueous solution of hydroxypropylmethyl cellulose) and dried. The granules were coated by spraying a 5 wt% solution prepared by dissolving hydroxypropylmethyl cellulose phthalate in a 1:1 mixture of ethanol and methylene chloride, and finally mixed with magnesium stearate using a double cone mixer.

### 2) Preparation of lovastatin layer

Predetermined amounts of lovastatin, microcrystalline cellulose and mannitol as shown in Table 3 were sieved with a No. 35 sieve and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60 °C using a steam dryer and ground with a No. 20 sieve. The sized granules were mixed with butylatedhydroxyanisole, sodium starch glycolate and colloidal silicon dioxide and finally mixed with magnesium stearate using a double cone mixer.

### 3) Compression and coating

Compression was performed using a compressor for the production of multi-layered tablet (MRC-37T: Sejong). In detail, the composition comprising simvastatin was input in a first power inlet and the composition comprising amlodipine was input in a second inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

### Example 12: Preparation of amlodipine-lovastatin multi-layered tablets

### 1) Preparation of amlodipine controlled-release layer

Predetermined amounts of amlodipine and microcrystalline cellulose as shown in Table 3 were sieved with a No. 35 sieve and mixed using a double cone mixer. The mixture was introduced into a high-speed mixer, combined by adding Kollicoat SR30D and granulated using an oscillator with a No. 20 sieve. The granules were dried at 60 °C using a steam dryer and tabulated with a No. 20 sieve. The sized granules were finally mixed with magnesium stearate using a double cone mixer.

### 2) Preparation of lovastatin layer

Predetermined amounts of lovastatin, microcrystalline cellulose and mannitol as shown in Table 3 were sieved with a No. 35 sieve and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60 °C using a steam dryer and ground with a No. 20 sieve. The sized granules were mixed with butylatedhydroxyanisole, sodium starch glycolate and colloidal silicon dioxide and finally mixed with magnesium stearate using a double cone mixer.

### 3) Compression and coating

Compression was performed using a compressor for the production of multi-layered tablet (MRC-37T: Sejong). In detail, the composition comprising lovastatin was input in a first power inlet and the composition comprising amlodipine was input in a second inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

### Example 13: Preparation of amlodipine-atorvastatin multi-layered tablets

### 1) Preparation of amlodipine controlled-release layer

Predetermined amounts of amlodipine and microcrystalline cellulose as shown in Table 3 were sieved with a No. 35 sieve and mixed using a double cone mixer. The mixture was introduced into a fluidized-bed granulator (GPCG 1: Glatt), granulated by spraying a binder solution (an aqueous solution of hydroxypropylmethyl cellulose) and dried. The granules were coated by spraying a 5 wt% solution prepared by dissolving hydroxypropylmethyl cellulose phthalate in a 1:1 mixture of ethanol and methylene chloride. The coated granules were finally mixed with magnesium stearate using a double cone mixer.

### 2) Preparation of atorvastatin layer

Predetermined amounts of atorvastatin, microcrystalline cellulose and mannitol as shown in Table 3 were sieved with a No. 35 sieve and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60°C using a steam dryer and ground with a No. 20 sieve. The sized granules were mixed with butylatedhydroxyanisole, sodium starch glycolate and colloidal silicon dioxide and finally mixed with magnesium stearate using a double cone mixer.

### 3) Compression and coating

Compression was performed using a compressor for the production of multi-layered tablet (MRC-37T: Sejong). In detail, the composition comprising atorvastatin was input in a first power inlet and the composition comprising amlodipine was input in a second inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

### Example 14: Preparation of amlodipine-atorvastatin multi-layered tablets

### 1) Preparation of amlodipine controlled-release layer

Predetermined amounts of amlodipine and microcrystalline cellulose as shown in Table 3 were sieved with a No. 35 sieve and mixed using a double cone mixer. The mixture was introduced into a high-speed mixer, combined by adding Kollicoat SR30D and granulated using an oscillator with a No. 20 sieve. The granules were dried at 60 °C using a steam dryer and ground with a No. 20 sieve. The sized granules were mixed with magnesium stearate using a double cone mixer.

### 2) Preparation of atorvastatin layer

Predetermined amounts of atorvastatin, microcrystalline cellulose and mannitol as shown in Table 3 were sieved with a No. 35 sieve and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60°C using a steam dryer and ground with a No. 20 sieve. The sized granules were mixed with butylatedhydroxyanisole, sodium starch glycolate and colloidal silicon dioxide and finally mixed with magnesium stearate using a double cone mixer.

### 3) Compression and coating

Compression was performed using a compressor for the production of multi-layered tablet (MRC-37T: Sejong). In detail, the composition comprising atorvastatin was input in a first power inlet and the composition comprising amlodipine was input in a second inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered controlled-release tablets.

### Example 15: Preparation of amlodipine-atorvastatin inner core tablets

### 1) Preparation of amlodipine core tablet

Predetermined amounts of amlodipine and microcrystalline cellulose as shown in Table 3 were sieved with a No. 35 sieve and mixed using a double cone mixer. The mixture were introduced into a fluidized-bed granulator (GPCG 1: Glatt), granulated by spraying a binder solution (an aqueous solution of hydroxypropylmethyl cellulose) and dried. The granules were added with carbomer 71G powders, and mixed with magnesium stearate with a double cone mixer. The resulting mixture was compressed using a rotary compressor (MRC-33: Sejong) at a speed of 30 rev olutions per minute(rpm) to provide tablets with a hardness of 7-9 kp, a thickness of 3.0 mm and a diameter of 5.5 mm. These tablets were used as core tablets.

### 2) Preparation of atorvastatin layer

Predetermined amounts of atorvastatin, microcrystalline cellulose and mannitol as shown in Table 3 were sieved with a No. 35 sieve and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60 °C using a steam dryer and ground with a No. 20 sieve. The sized granules were mixed with butylatedhydroxyanisole, sodium starch glycolate and colloidal silicon dioxide and finally mixed with magnesium stearate using a double cone mixer.

### 3) Compression and coating

Inner core tablets were prepared with a compressor for the production of inner core tablet (RUD-1: Kilian) by using the amlodipine core tablet and the composition containing atorvastatin as an inner core and an outer layer, respectively. The compression was performed at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea).

### Example 16: Preparation of lercanidipine-simvastatin multi-layered tablets

### 1) Preparation of lercanidipine controlled-release layer

Predetermined amounts of lercanidipine and microcrystalline cellulose as shown in Table 3 were sieved with a No. 35 sieve and mixed using a double cone mixer. The mixture was placed into a fluidized-bed granulator (GPCG 1: Glatt), and sprayed with a binder solution (an aqueous solution of hydroxypropylmethyl cellulose) to prepare granules, and dried. The granules were coated by spraying a 5 wt% solution prepared by dissolving hydroxypropylmethyl cellulose phthalate in a 1:1 mixture of ethanol and methylene chloride. The coated granules were finally mixed with magnesium stearate using a double cone mixer.

### 2) Preparation of simvastatin layer

Predetermined amounts of simvastatin, microcrystalline cellulose and mannitol as shown in Table 3 were sieved with a No. 35 sieve and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60 °C using a steam dryer and ground with a No. 20 sieve. The sized granules were mixed with butylatedhydroxyanisole, sodium starch glycolate and colloidal silicon dioxide and finally mixed with magnesium stearate using a double cone mixer.

### 3) Compression and coating

Compression was performed using a compressor for the production of multi-layered tablet (MRC-37T: Sejong). In detail, the composition comprising simvastatin was introduced into a first powder inlet, and then the composition comprising lercanidipine was introduced into a second powder inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

### Example 17: Preparation of lercanidipine-simvastatin multi-layered tablets

### 1) Preparation of lercanidipine controlled-release layer

Predetermined amounts of lercanidipine and microcrystalline cellulose as shown in Table 3 were sieved with a No. 35 sieve and mixed using a double cone mixer. The mixture was introduced into a high-speed mixer, combined by adding Kollicoat SR30D and granulated using an oscillator with a No. 20 sieve. The granules were dried at 60 °C using a steam dryer and ground with a No. 20 sieve. The sized granules were mixed with magnesium stearate using a double cone mixer.

### 2) Preparation of simvastatin layer

Predetermined amounts of simvastatin, microcrystalline cellulose and mannitol as shown in Table 3 were sieved with a No. 35 sieve and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60 °C using a steam dryer and ground with a No. 20 sieve. The sized granules were mixed with butylatedhydroxyanisole, sodium starch glycolate and colloidal silicon dioxide, and finally mixed with magnesium stearate using a double cone mixer.

### 3) Compression and coating

Compression was performed using a compressor for the production of multi-layered tablet (MRC-37T: Sejong). In detail, the composition comprising simvastatin was introduced into a first powder inlet, and the composition comprising lacidipine was introduced into a second powder inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

### Example 18: Preparation of lacidipine-simvastatin multi-layered tablets

### 1) Preparation of lacidipine controlled-release layer

Predetermined amounts of lacidipine and microcrystalline cellulose as shown in Table 3 were sieved with a No. 35 sieve and mixed using a double cone mixer. The mixture was introduced into a fluidized-bed granulator (GPCG 1: Glatt), granulated by spraying a binder solution (an aqueous solution, of hydroxypropylmethyl cellulose) and dried. The granules were coated by spraying a 5 wt% solution prepared by dissolving hydroxypropylmethyl cellulose phthalate in a 1:1 mixture of ethanol and methylene chloride, and finally mixed with magnesium stearate using a double cone mixer.

### 2) Preparation of simvastatin layer

Predetermined amounts of simvastatin, microcrystalline cellulose and mannitol as shown in Table 3 were sieved with a No. 35 sieve and mixed using a high-speed mixer. A binder solution prepared by dissolving hydroxypropyl cellulose and citric acid in water was combined with a mixture of main ingredients in a high-speed mixer and granulated using an oscillator with a No. 20 sieve. The granules were dried at 60 °C using a steam dryer and ground with a No. 20 sieve. The sized granules were mixed with butylatedhydroxyanisole, sodium starch glycolate and colloidal silicon dioxide, and finally mixed with magnesium stearate using a double cone mixer.

### 3) Compression and coating

Compression was performed using a compressor for the production of multi-layered tablet (MRC-37T: Sejong). In detail, the composition comprising simvastatin was introduced into a first powder inlet, and the composition comprising lacidipine was introduced into a second powder inlet. The compression was performed under such a condition that the interlayer incorporation may be minimized at a speed of 30 revolutions per minute(rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

### Example 19: Preparation of lacidipine-simvastatin multi-layered tablets

### 1) Preparation of lacidipine controlled-release layer

Predetermined amounts of lacidipine and microcrystalline cellulose as shown in Table 3 were sieved with a No. 35 sieve, and mixed using a double cone mixer. The mixture was mixed using Kollicoat SR30D in a high-speed mixer. Thus obtained mixture was granulated using oscillator with a No. 20 sieve, dried at 60 °C using a steam dryer and sized with a No. 20 sieve. The coated granules were mixed with magnesium stearate using a double cone mixer.

### 2) Preparation of simvastatin layer

Predetermined amounts of simvastatin, microcrystalline cellulose and mannitol as shown in Table 3 were sieved with a No. 35 sieve and mixed using a high-speed mixer. A binder solution was prepared by dissolving hydroxypropyl cellulose and citric acid in water and combining with the mixture of the main ingredients. Thus obtained mixture was combined, granulated using an oscillator with a No. 20 sieve, dried at 60 °C using a steam dryer and ground with a No. 20 sieve. The granules were mixed with butylatedhydroxyanisole, sodium starch glycolate and colloidal silicon dioxide, and finally mixed with magnesium stearate using a double cone mixer.

### 3) Compression and coating

The composition was compressed using a compressor for the production of a multi-layered tablet (MRC-37: Sejong). In detail, the composition comprising simvastatin was input in a first power inlet and the composition comprising amlodipine was input in a second inlet. The compression was performed under such a condition that the interlayer incorporation may be minimized at a speed of 30 revolutions per minute (rpm) to provide tablets with a hardness of 7-9 kp, a thickness of 6.0 mm and a diameter of 9.5 mm. Film coating layer was formed on the compressed tablets using Hi-coater (SFC-30N, Sejong mechanics, Korea), thus producing multi-layered tablets.

**Table 2**

| Ingredients | | Amounts (mg/tablet) | | | | | | | | | |
|---|---|---|---|---|---|---|---|---|---|---|---|
| | | Examples | | | | | | | | | |
| | | 1 | 2 | 3 | 4 | 5 | 6 | 7 | 8 | 9 | 10 |
| Controlled -release layer | Amlodipine maleate | 6.42 | 6.42 | 6.42 | 6.42 | 6.42 | 6.42 | 6.42 | 6.42 | 6.42 | 6.42 |
| | Lercanidipine HCl | - | - | - | | - | - | - | - | - | - |
| | Lacidipine | - | - | - | - | - | - | - | - | - | - |
| | Microcrystalline cellulose | 88.58 | 81.58 | 81.58 | 87.83 | 80.83 | 80.83 | 87.83 | 80.83 | 88.58 | 81.58 |
| | Kollicoat SR30D¹⁾ | - | 12 | - | - | 12 | - | - | 12 | - | 12 |
| | Eudragit RS PO²⁾ | - | - | 10 | - | - | 10 | - | - | - | - |
| | Hydroxypropylmethyl cellulose | 2 | - | 2 | 2 | - | 2 | 2 | - | 2 | - |
| | Hydroxypropylmethyl cellulose phthalate | 3 | - | - | 3 | - | - | 3 | - | 3 | - |
| | Magnesium stearate | - | - | - | 0.75 | 0.75 | 0.75 | 0.75 | 0.75 | - | - |
| Immediate -release layer | Simvastatin | 20 | 20 | 20 | 20 | 20 | 20 | 20 | 20 | 20 | 20 |
| | Lovastatin | - | - | - | - | - | - | - | - - | - - | - |
| | Atorvastatin | - | - | - | - | - | - | - | - | - | - |
| | Microcrystalline cellulose | 57 | 57 | 57 | 57 | 57 | 57 | 57 | 57 | 57 | 57 |
| | D-mannitol | 112.46 | 112.46 | 112.46 | 112.46 | 112.46 | 112.46 | 112.46 | 112.46 | 112.46 | 112.46 |
| | Sodium starch glycolate | 1 | 1 | 1 | 1 | 1 | 1 | 1 | 1 | 1 | 1 |
| | Butylatedhydroxy-anisole | 0.04 | 0.04 | 0.04 | 0.04 | 0.04 | 0.04 | 0.04 | 0.04 | 0.04 | 0.04 |
| | Hydroxypropylmethyl cellulose | 5 | 5 | 5 | 5 | 5 | 5 | 5 | 5 | 5 | 5 |
| | Aerosil 200³⁾ | 1 | 1 | 1 | 1 | 1 | 1 | 1 | 1 | 1 | 1 |
| | Citric acid | 2 | 2 | 2 | 2 | 2 | 2 | 2 | 2 | 2 | 2 |
| | Magnesium stearate | 1.5 | 1.5 | 1.5 | 1.5 | 1.5 | 1.5 | 1.5 | 1.5 | 1.5 | 1.5 |
| Coating layer | Hydroxypropylmethyl cellulose 2910 | 2.6 | 2.6 | 2.6 | 2.6 | 2.6 | 2.6 | 2.6 | 2.6 | - | - |
| | Hydroxypropyl cellulose | 2.6 | 2.6 | 2.6 | 2.6 | 2.6 | 2.6 | 2.6 | 2.6 | - | - |
| | Titanium oxide | 2.3 | 23 | 2.3 | 2.3 | 23 | 23 | 2.3 | 2.3 | - | - |
| | Talc | 1.5 | 1.5 | 1.5 | 1.5 | 1.5 | 1.5 | 1.5 | 1.5 | - | - |
| | Ethanol | 64.8 | 64.8 | 64.8 | 64.8 | 64.8 | 64.8 | 64.8 | 64.8 | - | - |
| | Distilled water | 16.2 | 16.2 | 16.2 | 16.2 | 16.2 | 16.2 | 16.2 | 16.2 | - | - |
| Total | | 309 | 309 | 309 | 309 | 309 | 309 | 309 | 309 | 300 | 300 |
| 1) Kollicoat SR30D - Main ingredient: polyacetate 30% suspension (BASF) | | | | | | | | | | | |
| 2) Eudragit RS PO - Main ingredient: polymethacrylate copolymer (BASF) | | | | | | | | | | | |
| 3) Aerosil 200 - Main ingredient: colloidal silicon dioxide (Degussa) | | | | | | | | | | | |

**Table 3**

| Ingredients | | Amounts (mg/tablet) | | | | | | | |
|---|---|---|---|---|---|---|---|---|---|
| | | Examples | | | | | | | |
| | | 11 | 12 | 13 | 14 | 15 | 16 | 17 | 18 |
| Controlled -release layer | Amlodipine maleate | 6.42 | 6.42 | 6.42 | 6.42 | - | - | - | - |
| | Lercanidipine HCl | - | - | - | - | 10- | 10 | - | - |
| | Lacidipine | - | - | - | - | - | - | 4 | 4 |
| | Microcrystalline cellulose | 87.83 | 80.83 | 87.83 | 80.83 | 79.25 | 74.25 | 90.25 | 83.25 |
| | Kollicoat SR30D¹⁾ | - | 12 | - | 12 | - | 15 | - | 12 |
| | Eudragit RS PO²⁾ | - | - | - | - | - | - | - | - |
| | Hydroxypropylmethyl cellulose | 2 | - | 2 | - | 4 | - | 2 | - |
| | Hydroxypropylmethyl cellulose phthalate | 3 | - | 3 | - | 6 | - | 3 | - |
| | Magnesium stearate | 0.75 | 0.75 | 0.75 | 0.75 | 0.75 | 0.75 | 0.75 | 0.75 |
| Immediate -release layer | Simvastatin | - | - | - | - | 20 | 20 | 20 | 20 |
| | Lovastatin | 20 | 20 | - | - | - | - | - | - |
| | Atorvastatin | - | - | 20 | 20 | - | - | - | - |
| | Microcrystalline cellulose | 57 | 57 | 57 | 57 | 57 | 57 | 57 | 57 |
| | D-mannitol | 112.46 | 112.46 | 112.46 | 112.46 | 112.46 | 112.46 | 112.46 | 112.46 |
| | Sodium starch glycolate | 1 | 1 | 1 | 1 | 1 | 1 | 1 | 1 |
| | Butylatedhydroxy-anisole | 0.04 | 0.04 | 0.04 | 0.04 | 0.04 | 0.04 | 0.04 | 0.04 |
| | Hydroxypropylmethyl cellulose | 5 | 5 | 5 | 5 | 5 | 5 | 5 | 5 |
| | Aerosil 200³⁾ | 1 | 1 | 1 | 1 | 1 | 1 | 1 | 1 |
| | Citric acid | 2 | 2 | 2 | 2 | 2 | 2 | 2 | 2 |
| | Magnesium stearate | 1.5 | 1.5 | 1.5 | 1.5 | 1.5 | 1.5 | 1.5 | 1.5 |
| Coating layer | Hydroxymethyl cellulose 2910 | 2.6 | 2.6 | 2.6 | 2.6 | 2.6 | 2.6 | 2.6 | 2.6 |
| | Hydroxypropyl cellulose | 2.6 | 2.6 | 2.6 | 2.6 | 2.6 | 2.6 | 2.6 | 2.6 |
| | Titanium oxide | 2.3 | 2.3 | 23 | 23 | 2.3 | 2.3 | 2.3 | 2.3 |
| | Talc | 1.5 | 1.5 | 1.5 | 1.5 | 1.5 | 1.5 | 1.5 | 1.5 |
| | Ethanol | 64.8 | 64.8 | 64.8 | 64.8 | 64.8 | 64.8 | 64.8 | 64.8 |
| | Distilled water | 16.2 | 162 | 16.2 | 16.2 | 16.2 | 16.2 | 16.2 | 16.2 |
| Total | | 309 | 309 | 309 | 309 | 309 | 309 | 309 | 309 |
| 1) Kollicoat SR30D - Main ingredient polyacetate 30% suspension (BASF) | | | | | | | | | |
| 2) Eudragit RS PO - Main ingredient: polymethacrylate copolymer (BASF) | | | | | | | | | |
| 3) Aerosil 200 - Main ingredient: colloidal silicon dioxide (Degussa) | | | | | | | | | |

### Experimental Example 1: Comparative dissolution profile test

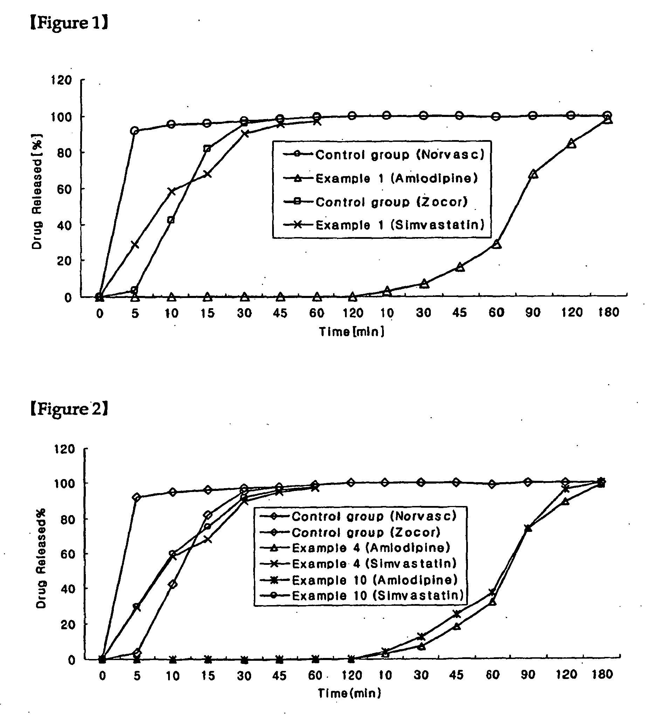

Comparative dissolution profile test was performed using an amlodipine/ simvastatin two-phase matrix tablets prepared in Example 1 and control drugs (Zocor^{®}: simvastatin single pill, MSD, Norvasc: amlodipine single pill, Pfizer). In the case of dissolution profile test of amlodipine ingredient, the dissolution solution was changed from an artificial gastric juice to an artificial intestinal juice after 2 hours. Specific process of dissolution profile test of each ingredient is described below, and the results are presented in Figure 1.

When the dissolution profile test was performed under the conditions described below, in the two-phase matrix tablets according to the present invention, the simvastatin ingredient showed a nearly equivalent dissolution behavior as compared to that of the control drug (Zocor^{®}), while the amlodipine ingredient showed a very delayed dissolution rate as compared to that of the control drug (Norvasc^{®}). In the case of the amlodipine/simvastatin two-phase matrix tablet according to the present invention, dissolution rates of amlodipine ingredient were all within 50% until one hour after the test began, which were far lower than that of the control drug (about 99%).

As described above, in the amlodipine/simvastatin two-phase matrix tablet according to the present invention, amlodipine shows a far lower initial dissolution rate than simvastatin unlike the control drugs (i.e., amlodipine single pill), and thus the amlodipine/simvastatin two-phase matrix tablet according to the present invention is less likely to be subject to the metabolism in liver ahead of simvastatin.

### Test method for amlodipine

Based on the general dissolution test method described in Korea Pharmacopoeia (8th revision)
Test method: Paddle method, 75 rpm
Dissolution solution: 0.01 M Hydrochloric acid, 750 mL
Analysis method: UV-visible spectrophotometry (detected wavelength = maximum 240 nm)

### Test method for simvastatin

Based on the 'Simvastatin tablet' part in USP X X IX
Test method: Paddle method, 50 rpm
Dissolution solution: pH=7.0 buffer solution (0.01 M monobasic sodium phosphate solution containing sodium lauryl sulfate 0.5% wt/wt as surfactant), 900 mL
Analysis method: UV-visible spectrophotometry (detected wavelength = maximum 247 nm and minimum 257 nm)

### Experimental Example 2: Comparative dissolution profile test

Comparative dissolution profile test was performed using an amlodipine/ simvastatin combined pharmaceutical formulation prepared in Examples 4 and 10 and control drugs (Zocor^{®}: simvastatin single pill, Norvasc^{®}: amlodipine single pill). The dissolution behavior of simvastatin and amlodipine was observed as described below, and in the case of dissolution profile test of amlodipine ingredient, the dissolution solution was changed from an artificial gastric juice to an artificial intestinal juice after 2 hours. Specific process of dissolution profile test of each ingredient is described below, and the results are presented in Figure 2.

When the dissolution profile test was performed under the conditions described below in Examples 4 and 10, the simvastatin ingredient showed a nearly equivalent dissolution behavior as compared to that of the control drug (Zocor^{®}), while the amlodipine ingredient showed a very delayed dissolution rate as compared to that of the control drug (Norvasc^{®}). In the case of the amlodipine/simvastatin multi-layered tablet according to the present invention, dissolution rates of amlodipine ingredient were all within 50% until one hour after the test began, which were far lower than that of the control drug (about 99%).

As described above, in the amlodipine/simvastatin multi-layered tablet according to the present invention, amlodipine shows a far lower initial dissolution rate than simvastatin unlike the control drugs (i.e., amlodipine single pill), and thus the amlodipine/simvastatin multi-layered tablet according to the present invention is less likely to be subject to the metabolism in liver ahead of simvastatin.

### Test method for amlodipine

Based on the general dissolution test method described in Korea Pharmacopoeia (8th revision)
Test method: Paddle method), 75 rpm
Dissolution solution: 0.01 M Hydrochloric acid, 750 mL
Analysis method: UV-visible spectrophotometry (detected wavelength = maximum 240 nm)

### Test method for simvastatin

Based on the 'Simvastatin tablet' part in USP X X IX
Test method: Paddle method, 50 rpm
Dissolution solution: pH=7.0 buffer solution (0.01 M monobasic sodium phosphate solution containing sodium lauryl sulfate 0.5% wt/wt as surfactant), 900 mL
Analysis method: UV-visible spectrophotometry (detected wavelength = maximum 247 nm and minimum 257 nm)

### Experimental Example 3: Comparative dissolution profile test

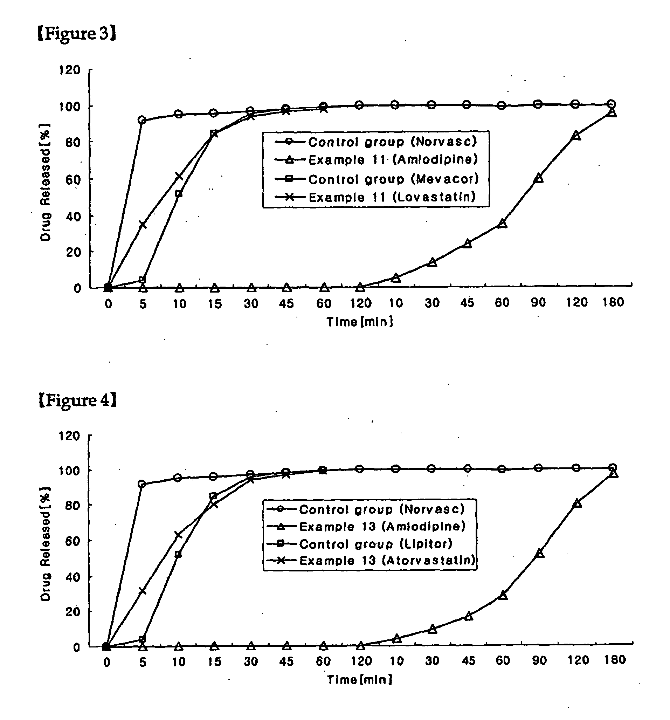

Comparative dissolution profile test was performed using an amlodipine/ lovastatin combined pharmaceutical formulation prepared in Example 11 and control drugs (Mevacor^{®}: lovastatin single pill, Norvasc^{®}: amlodipine single pill, Pfizer). In the case of dissolution profile test of amlodipine ingredient, the dissolution solution was changed from an artificial gastric juice to an artificial intestinal juice after 2 hours. Specific process of dissolution profile test of each ingredient is described below, and the results are presented in Figure 3.

When the dissolution profile test was performed under the conditions described below in Example 11, lovastatin ingredient showed a nearly equivalent dissolution behavior as compared to that of the control drug (Mevacor^{®}), while amlodipine ingredient showed a very delayed dissolution rate as compared to that of the control drug (Norvasc^{®}). In the case of the amlodipine/lovastatin multi-layered tablet according to the present invention, dissolution rates of amlodipine ingredient were all within 50% until one hour after the test began, which were far lower than that of the control drug (about 99%).

As described above, in the amlodipine/lovastatin multi-layered tablet according to the present invention, amlodipine shows a far lower initial dissolution rate than that of lovastatin unlike the control drugs (i.e., amlodipine single pill), and thus the amlodipine/lovastatin multi-layered tablet according to the present invention is less likely to be subject to the metabolism in liver ahead of lovastatin.

### Test method for amlodipine

Based on the general dissolution test method described in Korea Pharmacopoeia (8th revision)
Test method: Paddle method), 75 rpm
Dissolution solution: 0.01 M Hydrochloric acid, 750 mL
Analysis method: UV-visible spectrophotometry (detected wavelength = maximum 240 nm)

### Test method for lovastatin

Based on the 'Lovastatin tablet' part in USP X X IX
Test method: Paddle method, 50 rpm
Dissolution solution: pH=7.0 buffer solution (0.01 M monobasic sodium phosphate solution containing sodium lauryl sulfate 2.0% wt/wt as surfactant), 900 mL
Analysis method: High performance liquid chromatography
Detected wavelength: 230 nm
Mobile phase: Acetonitrile : 0.02 M monobasic sodium phosphate buffer solution (pH=4.0): methanol = 5: 3: 1
Column: Octadecyl silyl silica gel packed in a stainless steel tube of 4.6 mm (internal diameter) and 250 mm (length)
Flow rate: 1.5 mL/minute

### Experimental Example 4: Comparative dissolution profile test

Comparative dissolution profile test was performed using an amlodipine/atorvastatin combined pharmaceutical formulation prepared in Example 13 and control drugs (Lipitor^{®}: atorvastatin single pill, Norvasc: amlodipine single pill, Pfizer). In the case of dissolution profile test of amlodipine ingredient, the dissolution solution was changed from an artificial gastric juice to an artificial intestinal juice after 2 hours. Specific process of dissolution profile test of each ingredient is described below, and the results are presented in Figure 4.

When the dissolution profile test was performed under the conditions described below in Example 13, atorvastatin ingredient showed a nearly equivalent dissolution behavior as compared to that of the control drug (Lipitor^{®}), while amlodipine ingredient showed a very delayed dissolution rate as compared to that of the control drug (Norvasc^{®}). In the case of the amlodipine/atorvastatin multi-layered tablet according to the present invention, dissolution rates of amlodipine ingredient were all within 50% until one hour after the test began, which were far lower than that of the control drug (about 99%).

As described above, in the amlodipine/atorvastatin multi-layered tablet according to the present invention, amlodipine shows a far lower initial dissolution rate than atorvastatin unlike the control drugs (i.e., amlodipine single pill), and thus the amlodipine/atorvastatin multi-layered tablet according to the present invention is less likely to be subject to the metabolism in liver ahead of atorvastatin.

### Test method for amlodipine

Based on the general dissolution test method described in Korea Pharmacopoeia (8th revision)
Test method: Paddle method), 75 rpm
Dissolution solution: 0.01 M Hydrochloric acid, 750 mL
Analysis method: UV-visible spectrophotometry (detected wavelength = maximum 240 nm)

### Test method for atorvastatin

Based on the general dissolution test method described in Korea Pharmacopoeia (8th revision)
Test method: Paddle method, 50 rpm
Dissolution solution: pH=7.0 buffer solution (0.01 M monobasic sodium phosphate solution containing sodium lauryl sulfate 2% wt/wt as surfactant), 900 mL
Analysis method: High performance liquid chromatography
Detected wavelength: 247 nm
Mobile phase: Methanol: 0.025 M monobasic sodium phosphate buffer solution (pH=4.0): methanol = 67 : 33 (pH=4.0)
Column: Octadecyl silyl silica gel packed in a stainless steel tube of 4.6 mm (internal diameter) and 250 mm (length)
Flow rate: 1.5 mL/minute

### Experimental Example 5: Comparative dissolution profile test

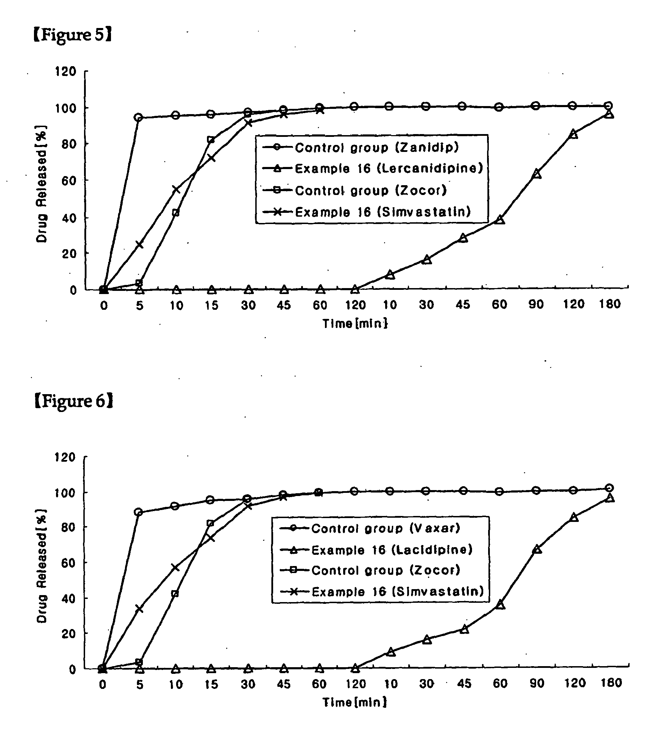

Comparative dissolution profile test was performed using a lercanidipine/ simvastatin combined pharmaceutical formulation prepared in Example 16 and control drugs (Zocor^{®}: simvastatin single pill, Zanidip^{®}: lercanidipine single pill, LG Life Sciences Ltd.). In the case of dissolution profile test of lercanidipine ingredient, the dissolution solution was changed from an artificial gastric juice to an artificial intestinal juice after 2 hours. Specific process of dissolution profile test of each ingredient is described below, and the results are presented in Figure 5.

When the dissolution profile test was performed under the conditions described below in Example 16, the simvastatin ingredient showed a nearly equivalent dissolution behavior as compared to that of the control drug (Zocor^{®}), while the lercanidipine ingredient showed a very delayed dissolution rate as compared to that of the control drug (Zanidip^{®}). In the case of the lercanidipine/simvastatin multi-layered tablet according to the present invention, dissolution rates of lercanidipine ingredient were all within 50% until one hour after the test began, which were far lower than that of the control drug (about 99%).

As described above, in the lercanidipine/simvastatin multi-layered tablet according to the present invention, lercanidipine shows a far lower initial dissolution rate than simvastatin unlike the control drugs (i.e., lercanidipine single pill), and thus the lercanidipine/simvastatin multi-layered tablet according to the present invention is less likely to be subject to the metabolism in liver ahead of simvastatin.

### Test method for lercanidipine

Based on the general dissolution test method described in Korea Pharmacopoeia (8th revision)
Test method: Paddle method), 75 rpm
Dissolution solution: 0.01 M Hydrochloric acid, 750 mL
Analysis method: High performance liquid chromatography
Detected wavelength: 356 nm
Mobile phase: Acetonitrile: 0.01 M phosphate buffer solution = 45 : 55 (pH=4.0)
Column: Octadecyl silyl silica gel packed in a stainless steel tube of 4.6 mm (internal diameter) and 250 mm (length)
Flow rate: 1.0 mL/minute

### Test method for simvastatin

Based on the 'Simvastatin tablet' part in USP X X IX
Test method: Paddle method, 50 rpm
Dissolution solution: pH=7.0 buffer solution (0.01 M monobasic sodium phosphate solution containing sodium lauryl sulfate 0.5% wt/wt as surfactant), 900 mL
Analysis method: UV-visible spectrophotometry (detected wavelength = maximum 247 nm and minimum 257 nm)

### Experimental Example 6: Comparative dissolution profile test

Comparative dissolution profile test was performed using a lacidipine/ simvastatin combined pharmaceutical formulation prepared in Example 18 and control drugs (Zocor^{®}: simvastatin single pill, MSD, Vaxar^{®}: lacidipine single pill, GlaxoSmithkline Plc.). In the case of dissolution profile test of lacidipine ingredient, the dissolution solution was changed from an artificial gastric juice to an artificial intestinal juice after 2 hours. Specific process of dissolution profile test of each ingredient is described below, and the results are presented in Figure 6.

When the dissolution profile test was performed under the conditions described below in Example 18, the simvastatin ingredient showed a nearly equivalent dissolution behavior as compared to that of the control drug (Zocor^{®}), while the lacidipine ingredient showed a very delayed dissolution rate as compared to that of the control drug (Vaxar^{®}). In the case of the lacidipine/simvastatin multi-layered tablet according to the present invention, dissolution rates of lacidipine ingredient were all within 50% until one hour after the test began, which were far lower than that of the control drug (about 99%).

As described above, in the lacidipine/simvastatin multi-layered tablet according to the present invention, lacidipine shows a far lower initial dissolution rate than simvastatin unlike the control drugs (i.e., lacidipine single pill), and thus the lacidipine/simvastatin multi-layered tablet according to the present invention is less likely to be subject to the metabolism in liver ahead of simvastatin.

### Test method for lacidipine

Based on the general dissolution test method described in Korea Pharmacopoeia (8th revision)
Test method: Paddle method), 75 rpm
Dissolution solution: 0.01 M Hydrochloric acid, 750 mL
Analysis method: High performance liquid chromatography
Detected wavelength: 282 nm
Mobile phase: Acetronitrile:0.05 M ammonium acetate buffer solution = 80:20
Column: Octadecyl silyl silica gel packed in a stainless steel tube of 4.6 mm (internal diameter) and 250 mm (length)
Flow rate: 1.0 mL/minute

### Test method for simvastatin

Based on the 'Simvastatin tablet' part in USP X X IX
Test method: Paddle method, 50 rpm
Dissolution solution: pH=7.0 buffer solution (0.01 M monobasic sodium phosphate solution containing sodium lauryl sulfate 0.5% wt/wt as surfactant), 900 mL
Analysis method: UV-visible spectrophotometry (detected wavelength = maximum 247 nm and minimum 257 nm)

### Experimental Example 7: Preliminary clinical test

Preliminary clinical test was conducted as described in Table 4 to compare the therapeutic effects of (i) a single administration of commercially available 'Zocor ^{®} tablet' (simvastatin 20 mg, MSD), (ii) simultaneous co-administration of Zocor^{®} tablet and 'Norvasc^{®} tablet' (amlodipine besylate 5 mg, Pfizer) and, as an experimental group, (iii) a chronotherapeutic administration of Zocor^{®} tablet and Norvasc^{®} tablet. The chronotherapeutic administration was so designed that the release time of drugs are the same as in a combined formulation according to the present invention.

**Table 4. Summary of the clinical test**

| | | | | |
|---|---|---|---|---|
| Title | Multicenter trial (academic, investigator initiated trial) for comparing the pharmacokinetic characteristics, efficacy and safety in (i) a simultaneous co-administration, (ii) a chronotherapeutic administration and (iii) a single administration of amlodipine and simvastatin to subjects with hypertension and hyperlipidemia | | | |
| Object | Comparative evaluation of steady-state pharmacokinetic characteristics, efficacy and safety in a simultaneous co-administration and a chronotherapeutic administration of amlodipine and simvastatin in the evening | | | |
| Test subject | Eight male and female adults aged 30-75 with hypertension and hyperlipidemia per each group (total 32 subjects) | | | |
| Test design | This clinical test is designed as follows. | | | |
| | 4open-label, single dose, randomized | | | |
| | - Drug 1: Norvasc^{®} 5 mg (single pill) | | | |
| | - Drug 2: Zocor^{®} 20 mg (single pill) | | | |
| | Twenty four subjects are divided into 3 groups, i.e., (i) a Zocor^{®} single administration group, (ii) a simultaneous co-administration group of Norvasc^{®} and Zocor^{®} in the evening and (iii) a chronotherapeutic administration group of Norvasc^{®} and Zocor^{®} in the evening. Each subject is administered for 6 weeks (42 days), and the pharmacokinetic characteristics, efficacy and safety of the three groups are compared. | | | |
| Evaluation | 1. Evaluation of efficacy | | | |
| | - Primary evaluation: Normalization of the mean systolic blood pressure measured with an automatic blood pressure meter and LDL-C are compared between a simultaneous co-administration group and a chronotherapeutic administration group after the test is finished. | | | |
| | - Secondary evaluation: Normalization of the mean diastolic blood pressure measured with an automatic blood pressure meter, pulse pressure, lipid profiles (total cholesterol (mg/dl), LDL-cholesterol, (mg/dl), HDL-cholesterol (mg/dl) and triglyceride (mg/dl) are compared between a simultaneous co-administration group and a chronotherapeutic administration group after the test is finished. | | | |
| | | | | |
| | 2. Evaluation of drug concentration | | | |
| | Plasma concentrations of simvastatin, simvastatin acid and amlodipine are measured after administration by using LC/MS/MS. Four groups are compared in terms of the following parameters: | | | |
| | - Area under the plasma concentration-time curve: AUC_{0-∞}, AUC₀₋ₜ₂, AUC_{0-∞} | | | |
| | - Maximum plasma concentration: Cₘₐₓ | | | |
| | - Elimination half-life: T_{1/2} | | | |

| | | Group | Drug and administration | Subject number |
|---|---|---|---|---|
| Test group | | Chronotherapeutic administration | Simvastatin (20 mg) at 7 p.m. Amlodipine (5 mg) at 10-11 p.m. | 8 |
| | | Simultaneous administration | Simvastatin (20 mg) and amlodipine (5 mg) at 7 p.m. | 8 |
| | | Simvastatin single administration | Simvastatin (20 mg) at 7 p.m. | 8 |

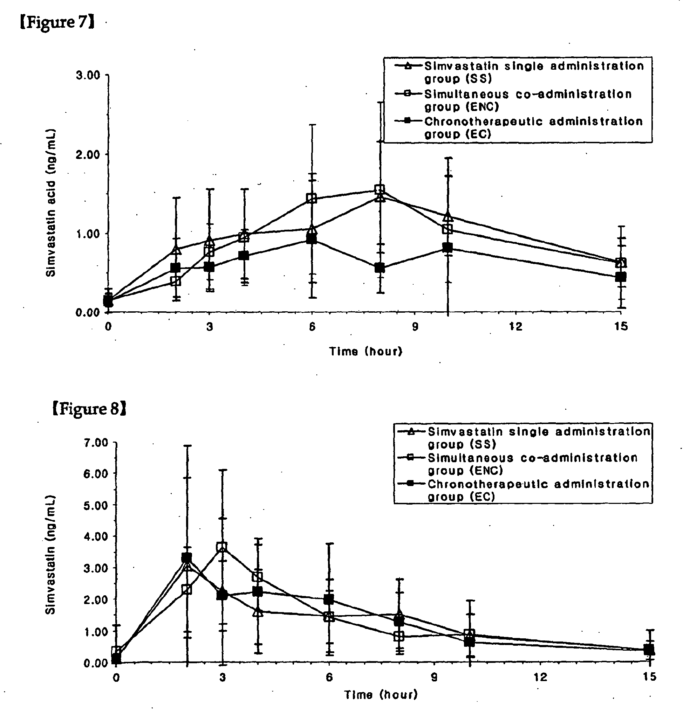

This test was conducted for ascertaining the effect of the present invention by using the standards of domestic and foreign clinical test for the medication approval and reducing the numbers of experimental groups. However, test subjects were strictly controlled, and the standards of clinical test were rigidly followed during the test. Comparative results of the clinical test are presented in Table 5 and Figures 7-10.

**Table 5. Pharmacokinetic/pharmacodynamic results of the clinical test**

| Clinical test for comparing a chronotherapeutic administration and a simultaneous co-administration (Korea University Medical Center) | | | | |
|---|---|---|---|---|
| 1 | Disease | Hypertension with hyperlipidemia | Hypertension with hyperlipidemia | Hyperlipidemia |
| 2 | Group | Chronotherapeutic administration group (EC) | Simultaneous co-administration group (ENC) | Simvastatin single administration group (SS) |
| 3 | Number of subject | 7 | 9 | 7 |
| | | After daily administration for 41 days | | |
| 4 | Systolic blood pressure | 121 mmHg | 127 mmHg | - |
| 5 | Diastolic blood pressure | 80 mmHg | 82 mmHg | - |
| 6 | Mean blood pressure | 94mmHg | 97mmHg | - |
| 7 | Pulse pressure | 40 mmHg | 45 mmHg | - |
| 8 | Total cholesterol | 158 mg/dl | 164 mg/dl | 170 mg/dl |
| 9 | LDL | 88 mg/dl | 94 mg/dl | 95 mg/dl |
| 10 | Neutral lipid | 107 mg/dl | 143 mg/dl | 134 mg/dl |
| 11 | HDL | 61 mg/dl | 46 mg/dl | 49 mg/dl |
| 12 | S-GPT | 23 IU/L | 39 IU/L | 31 IU/L |
| 13 | S-GOT | 23 IU/ L | 38 IU/L | 33 IU/L |
| 14 | CPK | 71 IU/L | 85 IU/L | 117 IU/L |
| 15 | r-GPT | 38 IU/ L | 46 IU/L | 28 IU/L |
| 16 | Alkaline Phosphatase | 70IU/L (No significant change) | 57 IU/L (No significant change) | 59 IU/L (No significant change) |
| 17 | Clinical adverse effect | One case (Diarrhea) | One case (Fatigue) One case (Cold) | - |
| 18 | Simvastatin acid AUC (ng·hr/mL) | 14.21 | 23.4 | 24.59 |
| 19 | Simvastatin AUC (ng·hr/mL) | 24.29 | 20.29 | 21.62 |
| 20 | Simvastatin + Simvastatin acid AUC (ng·hr/mL) | 38.50 | 43.73 | 46.21 |
| 21 | Amlodipine AUC₀₋₁₅ (ng·hr/mL) | 111.38 | 107.11 | - |

1. The chronotherapeutic administration group showed the lowest systolic and diastolic blood pressure on the 41st day of daily administration.
2. The chronotherapeutic administration group showed lower total cholesterol and low density lipoprotein (LDL) cholesterol on the 41st day of daily administration than the simultaneous co-administration group and the simvastatin single administration group.
3. The chronotherapeutic administration group showed the highest activity of lowering the triglyceride, the pathogenically important lipid and the simvastatin single administration group was the second in lowering the triglyceride and the simultaneous co-administration group showed no activity of lowering triglyceride. Although a statin-based agent is an agent that inhibits the synthesis of cholesterol, it is known to inhibit the synthesis of triglyceride by 8-25% because the synthesis of cholesterol is a reaction rate determining step in the synthesis of lipoprotein. The chronotherapeutic administration group and the simvastatin single administration group lowers the synthesis of triglyceride by 20% and 10%, respectively, and the simultaneous co-administration group showed no activity of lowering the synthesis of neutral lipid. One reason could be that the unnecessary higher blood concentration of simvastatin by about 30% in the simultaneous co-administration group due to the antagonistic effects incurred by amlodipine on the hepatic enzymes in liver seemed also to result in the lower control on the synthesis of triglyceride. Meanwhile, the chronotherapeutic administration group showed lower synthesis of lipid than the simvastatin single administration. This is due to the lipid-lowering activity of amlodipine.
4. It is well known that HDL value should be increased for improving lipid-related diseases. The chronotherapeutic administration group showed the highest increase in HDL value.
5. Safety of drug formulation was evaluated by observing change in biomarkers on the 41st day of daily administration, and the results are as follows.
1) The chronotherapeutic administration showed the most favorable safety biomarkers in terms of S-GPT, S-GOT, CPK, γ-GPT and alkaline phosphatase values.
2) The simultaneous co-administration group was higher than the chronotherapeutic administration group in the concentration of simvastatin in plasma by more than 30%, causing unnecessary inflammation caused by simvastatin.

6. Only two subjects showed three cases of side effects in the total three groups. Only one case of diarrhea was observed in the chronotherapeutic administration group, and two cases (fatigue and cough) were observed in the simultaneous co-administration group. Plasma concentration of drugs was changed as follows.
1) Plasma concentration of simvastatin acid:
The chronotherapeutic administration was lower in plasma concentration of simvastatin acid by 40% than the simultaneous co-administration and the simvastatin single administration (Table. 6 and Figure 7). This pharmacokinetic data (table 6) shows that the chronotherapeutic administration is superior in lowering lipid to the simultaneous co-administration. (It implies that higher concentration of active simvastatin acids than inactive simvastatin are consumed in liver to inhibit cholesterol synthesis and are further completely metabolized in liver to be excreted through bile duct but do not entering into the blood)

**Table 6. Increase in plasma concentration of simvastatin acid**

| Simvastatin acid | AUC_{INF}/ng·hr/mL) | | Increase in plasma concentration (%) |
|---|---|---|---|
| | Average | Deviation | |
| Single administration of Simvastatin (SS) | 24.59 | 13.66 | - |
| Simultaneous co-administration (ENC) | 23.44 | 12.52 | -4.67 |
| Chronotherapeutic administration (EC) | 14.21 | 7.67 | -42.23 |

2) Plasma concentration of simvastatin: As shown in Table 7 and Figure 8, the three groups were similar in plasma concentration of simvastatin.

**Table 7. Increase in plasma concentration of simvastatin**

| Simvastatin | AUC_{INF}(ng·hr/mL) | | Increase in plasma concentration (%) |
|---|---|---|---|
| | Average | Deviation | |
| Single administration of Simvastatin (SS) | 21.62 | 16.93 | - |
| Simultaneous co-administration (ENC) | 20.29 | 10.40 | -6.13 |
| Chronotherapeutic administration (EC) | 24.29 | 5.74 | 12.39 |

3) Total plasma concentration of simvastatin and simvastatin acids as provided in Table 8 shows that chronotherapeutic administration has lowest blood concentration of total simvastatins, meaning the highest concentration of them is consumed in liver and therefore shows the most effective lipid lowering figures and better biomarkers (to judge the possibility of side effect).

**Table 8. Increase in total plasma concentration of simvastatin acid and simvastatin**

| Simvastatin + Simvastatin acid | AUC_{INF}(ng·hr/mL) | | Increase in plasma concentration (%) |
|---|---|---|---|
| | Average | Deviation | |
| Single administration of Simvastatin (SS) | 46.21 | 30.58 | - |
| Simultaneous co-administration (ENC) | 43.74 | 22.92 | -5.36 |
| Chronotherapeutic administration (EC) | 38.50 | 13.40 | -16.68 |

4) Plasma concentration of amlodipine (Table 9): Plasma concentration of amlodipine is closely related to the activity of lowering blood pressure, and also to a lipid lowering activity because amlodipine has indirect activity in lowering lipid by improving atherosclerosis.

**Table 9. Plasma concentration of amlodipine**

| Amlodipine | AUC₀₋₁₅(ng·hr/mL) | |
|---|---|---|
| | Average | Deviation |
| Simultaneous co-administration (ENC) | 107.11 | 20.38 |
| Chronotherapeutic administration (EC) | 111.38 | 17.73 |

As shown in Figures 7-9, the chronotherapeutic administration with time interval (Simvastatin first dissolved and absorbed in liver and then 3-4 hours later amlodipine following the same course) makes it possible that simvastatin acids as active forms could be fully formed from inactive simvastatin by the hepatic enzyme Cytochrome P450 3A4 and fully utilized in liver and then further metabolized by the same hepatic enzymes to be excreted through the bile duct.

Therefore not showing unnecessary higher blood concentration of simvastatin acids as shown in the simultaneous co-administration group, where the hepatic same enzymes Cytochrome P450 3A4 are inhibited to be induced by amlodipine at the presence of simvastatin in liver, therefore the metabolism of simvastatin is partial, some of the formed simvastatin acids are not further metabolized after acting. And then flow into the blood to cause the unnecessary high blood concentration of simvastatin acids, which could be the source of developing side effect, such muscular disorder like rhabdomyolysis.

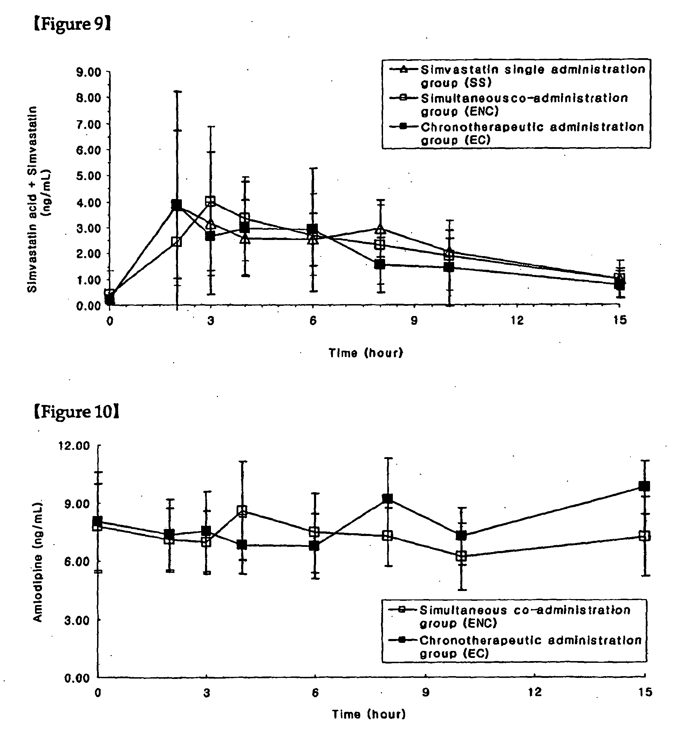

Further, as shown in Figure 10, Tₘₐₓ (time to reach maximum plasma concentration) of a dihydropyridine, calcium channel blocker represented by amlodipine was delayed for about 4-5 hours in the chronotherapeutic administration group, and the dihydropyridine, calcium channel blocker shows a delayed activity of lowering blood pressure as if administered at 12 in the evening. As a result, unlike a normal simultaneous co-administration group, a chronotherapeutic combined formulation may have a maximized activity of lowering blood pressure during the time between the morning and the noon next day, when mean blood pressure is highest.

As described above, the chronotherapeutic administration, designed as a combined formulation herein of a dihydropyridine, calcium channel blocker and a statin, a lipid-lowering agent, inhibits the side effects of a statin, lipid-lowering agent as compared to the simultaneous co-administration. The chronotherapeutic administration herein also enables a dihydropyridine, calcium channel blocker, to demonstrate its maximized clinical effects in lowering blood pressure.

Table 10 compares the chronotherapeutic administration with the simultaneous co-administration in lipid lowering efficacy. The chronotherapeutic administration group of Norvasc tablet (amlodipine besylate 5 mg) and Zocor tablet (simvastatin 20 mg) to be taken with time interval in the evening more remarkably decrease the plasma concentration of LDL-cholesterol and total cholesterol than the simultaneous co-administration group.
As ascertained in Figures 7-9, the combination products of the present invention may well be expected to demonstrate the same result that the drug component simvastatin transforms into an activated form in liver by hepatic enzyme cytochrome P450 and fully exerts pharmacological action in liver and fully metabolized by the same hepatic enzyme to be excreted through the bile duct, without being antagonistically affected by amlodipine.

**Table 10. Total cholesterol, HDL, LDL and neutral lipid (* p < 0.05, ** p < 0.01 vs. screening)**

| Group | | Chronotherapeutic administration group (EC) | Simultaneous co-administration group (ENC) | Zocor single administration group (SS) |
|---|---|---|---|---|
| Total cholesterol | Screening | 225 ± 28.0 | 239 ± 52.9 | 260 ± 50.7 |
| | D41 | 158 ± 25.1 | 164 ± 32.9 | 170 ± 18.9 |
| HDL | Screening | 61 ± 18.0 | 48 ± 13.6 | 52 ± 12.7 |
| | D41 | 61 ± 20.5 | 46 ± 7.6 | 49 ± 7.3 |
| LDL | Screening | 153 ± 14.6 | 161 ± 43.1 | 180 ± 44.2 |
| | D41 | 88 ± 11.8 | 94 ± 1.0 | 95 ± 24.6 |
| Triglyceride | Screening | 135 ± 69.3 | 150 ± 57.3 | 160 ± 72.4 |
| | D41 | 107 ± 29.1 | 143 ± 65.2 | 134 ± 39.0 |

Tables 11 and 12 show the results relating to blood pressure and pulse pressure, respectively, of the simultaneous co-administration group and the chronotherapeutic administration group. This ascertains that mean sitting systolic blood pressure and mean sitting diastolic blood pressure are significantly reduced in chronotherapeutic administration group. It surely is due to the high blood concentration of amlodipine (Figure 10) in chronotherapeutic administration group.

As a result, the chronotherapeutic administration group shows superiority compared to the simultaneous co-administration group in lowering blood pressure by delaying the release of amlodipine for about 4 hours.

**Table 11. Blood pressure (* p < 0.05, ** p < 0.01 vs. screening)**

| Group | | Chronotherapeutic administration group (EC) | Simultaneous co-administration group (ENC) |
|---|---|---|---|
| Systolic blood pressure (mmHg) | Screening | 148 ± 6.3 | 150 ± 5.9 |
| | D41 | 121 ± 8.8 | 127 ± 7.1 |
| | D42 | 117 ± 10.2 | 125 ± 8.8 |
| Diastolic blood pressure (mmHg) | Screening | 95 ± 4.0 | 95 ± 2.8 |
| | D41 | 80 ± 5.4 | 82 ± 8.1 |
| | D42 | 74 ± 8.2 | 79 ± 7.3 |
| Mean blood pressure (mmHg) | Screening | 113 ± 4.5 | 113 ± 3.6 |
| | D41 | 94 ± 5.8 | 97 ± 7.1 |
| | D42 | 88 ± 8.6 | 94 ± 7.5 |

**Table 12. Pulse pressure and pulse frequency (* p < 0.05, ** p < 0.01 vs. screening)**

| Group | | Chronotherapeutic administration group (EC) | Simultaneous co-administration group (ENC) |
|---|---|---|---|
| Pulse pressure (mmHg) | Screening | 53 ± 4.3 | 55 ± 4.2 |
| | D41 | 40 ± 7.1 | 45 ± 7.0 |
| | D42 | 43 ± 4.6 | 46 ± 4.6 |
| Pulse frequency (bpm) | Screening | 75 ± 16.2 | 63 ± 10.1 |
| | D41 | 73 ± 14.8 | 72 ± 11.5 |
| | D42 | 76 ± 12.5 | 68 ± 10.5 |

In conclusion, the clinical test above ascertains that chronotherpeutically combined formulation of a dihydropyridine, calcium channel blocker and a statin, lipid-lowering agent according to the present invention shall synergistically demonstrate remarkable improvement in inhibiting high cholesterol by a statin, lipid-lowering agent and lowering blood pressure by a dihydropyridine, calcium channel blocker at the same dosage as compared to a simultaneous co-administration of the two drugs, by means of the effects of release time control of each drug components.

### [Industrial applicability]

As mentioned above, the present invention permits the combination product of two drugs to demonstrate chronotherapeutic effects and synergistic effects in improving efficacy in better way and safety in less way. Accordingly the control release formulation of the present invention is based on theory of xenobiotics and chronotherapy.

The formulation of the present invention comprises as active ingredients, statin, a lipid-lowering agent, and a dihydropyridine, a calcium channel blocker, both of which are affected by to the same cytochrome P450 enzyme, in such a manner that one is affecting on while the other is affected by that enzyme, and is characterized in that the release rates of the aforementioned ingredients are different and the dissolution and absorption of each drug are initiated at certain intervals of time in a controlled manner. As a result, the formulation of the present invention is more useful pharmacologically, clinically, scientifically and economically in the treatment of a chronical circulatory disorder than when the two drugs are simultaneously co-administered without definitive time interval without consideration of the chronotherapeutic rhythm pattern.

The combination pharmaceutical formulation of the present invention causes drugs to be released at different rates, thereby preventing the antagonistic effects and lessening side effects, while maintaining the synergistic effect of the drugs. Furthermore, the combination pharmaceutical formulation of the present invention is administered once a day as a single dose, thus providing convenience in medication and saving time of prescribers for patient instruction.

## Claims

1. A chronotherapeutic combination pharmaceutical formulation with controlled-release comprising a controlled-release part containing a dihydropyridine, a calcium channel blocker, and an immediate-release part containing statin, a lipid-lowering agent, as active ingredients.

2. The chronotherapeutic combination pharmaceutical formulation of claim 1, wherein the release property of the dihydropyridine, calcium channel blocker and the statin, lipid-lowering agent are controlled so that the dihydropyridine, calcium channel blocker can be dissolved to be absorbed in the liver 3-4 hours later than the statin, lipid-lowering agent.

3. The chronotherapeutic combination pharmaceutical formulation of claim 1, wherein the release properties of the dihydropyridine, calcium channel blocker and the statin, lipid-lowering agent are controlled to decrease the interaction between the two drug ingredients metabolized at the same time in the liver via cytochrome P450 such that the dihydropyridine, calcium channel blocker can be released 2 hours after the onset of the release of the statin, lipid-lowering agent.

4. The chronotherapeutic combination pharmaceutical formulation of claim 1, wherein the dihydropyridine, calcium channel blocker is selected from the group consisting of amlodipine, lercanidipine, lacidipine and a pharmaceutically acceptable salt thereof; optionally wherein the dihydropyridine, calcium channel blocker is
(a) amlodipine and/or a pharmaceutically acceptable salt thereof or an isomer thereof; or
(b) amlodipine maleate or amlodipine besylate.

5. The chronotherapeutic combination pharmaceutical formulation of claim 1, wherein the formulation comprises 1-20 mg of dihydropyridine, calcium channel blocker.

6. The chronotherapeutic combination pharmaceutical formulation of claim 1, wherein the controlled-release part comprises a release controlling material selected from the group consisting of an enteric polymer, a water-insoluble polymer, a hydrophobic compound, a hydrophilic non-polymeric compound and a hydrophilic polymer; optionally wherein
(a) the release controlling material in the controlled-release part is contained in an amount of 10-500 weight parts relative to 100 weight parts of the dihydropyridine, a calcium channel blocker;
(b) the enteric polymer is selected from the group consisting of polyvinylacetate phthalate, methacrylic acid copolymer, hydroxypropylmethyl cellulose phthalate, shellac, cellulose acetate phthalate, cellulose propionate phthalate, Eudragit L, Eudragit S and a mixture thereof;
(c) the water-insoluble polymer is polyvinylacetate, methacrylic acid copolymer selected from the group consisting of poly(ethylacrylate-co-methylmethacrylate) copolymer or poly(ethylacrylate-methyl methacrylate-trimethyl aminoethyl methacrylate) copolymer, ethyl cellulose, cellulose acetate and a mixture thereof;
(d) the hydrophobic compound is selected from the group consisting of a fatty acid and a fatty acid ester, a fatty acid alcohol, a wax, an inorganic material and a mixture thereof; or
(e) each of the hydrophilic non-polymeric compound and the hydrophilic polymer is selected from the group consisting of a saccharide, a cellulose derivative, a gum, a protein, a polyvinyl derivative, a polymethacrylate copolymer, a polyethylene derivative, a carboxyvinyl polymer and a mixture thereof.

7. The chronotherapeutic combination pharmaceutical formulation of claim 6(d), wherein the fatty acid and fatty acid esters are selected from the group consisting of glyceryl palmitostearate, glyceryl stearate, glyceryl behenate, cetyl palmitate, glyceryl mono oleate, stearic acid and a mixture thereof; the fatty acid alcohol is selected from the group consisting of cetostearyl alcohol, cetyl alcohol, stearyl alcohol and a mixture thereof; the wax is selected from the group consisting of Carnauba wax, beeswax, microcrystalline wax and a mixture thereof; the inorganic material is selected from the group consisting of talc, precipitated calcium carbonate, dibasic calcium phosphate, zinc oxide, titanium oxide, kaolin, bentonite, montmorillonite, veegum and a mixture thereof.

8. The chronotherapeutic combination pharmaceutical formulation of claim 6(e), wherein the saccharide is selected from the group consisting of dextrin, polydextrin, dextran, pectin and pectin derivative, alginate, poly(galacturonic acid), xylan, arabinoxylan, arabinogalactan, starch, hydroxypropyl starch, amylose, amylopectin and a mixture thereof; wherein
the cellulose derivative is selected from the group consisting of hydroxypropylmethyl cellulose, hydroxypropyl cellulose, hydroxymethyl cellulose, hydroxyethyl cellulose, methyl cellulose, carboxymethyl cellulose sodium, hydroxypropyl methyl cellulose acetate succinate, hydroxyethylmethyl cellulose and a mixture thereof;
the gum is selected from the group consisting of guar gum, locust bean gum, tragacantha, carrageenan, gum acasia, gum arabic, gellan gum, xanthan gum and a mixture thereof;
the protein is selected from the group consisting of gelatin, casein, zein and a mixture thereof;
the polyvinyl derivative is selected from the group consisting of polyvinyl alcohol, polyvinyl pyrrolidone, poly(vinylacetal diethylaminoacetate) and a mixture thereof;
the polymethacrylate copolymer is selected from the group consisting of a **poly(butyl methacrylate**-(2-dimethylaminoethyl) methacrylate-methyl methacrylate) copolymer, a poly(methacrylic acid-methyl methacrylate) copolymer, a poly(methacrylic acid-ethyl acrylate) copolymer and a mixture thereof;
the polyethylene derivative is selected from the group consisting of polyethylene glycol, polyethylene oxide and a mixture thereof; and
the carboxyvinyl polymer is carbomer.

9. The chronotherapeutic combination pharmaceutical formulation of claim 1, wherein the statin, a lipid-lowering agent, is selected from the group consisting of simvastatin, lovastatin, atorvastatin, pitavastatin, rosuvastatin, fluvastatin, pravastatin and a mixture thereof; and optionally wherein the statin, a lipid-lowering agent, is selected from the group consisting of simvastatin, lovastatin, atorvastatin and a mixture thereof.

10. The chronotherapeutic combination pharmaceutical formulation of claim 1, wherein the statin, a lipid-lowering agent, is contained in an amount of 5-160 mg.

11. The chronotherapeutic combination pharmaceutical formulation of claim 1, which is a single pill with a structure of two-phased matrix, wherein the controlled-release part is placed discontinuously, thereby causing the controlled-release of the dihydropyridine, a calcium channel blocker, and the immediate-release part is placed continuously, thereby causing the immediate-release of the statin, a lipid-lowering agent.

12. The chronotherapeutic combination pharmaceutical formulation of claim 1, wherein the controlled-release part and the immediate-release part form a multi-layered structure.

13. The chronotherapeutic combination pharmaceutical formulation of claim 1, which is a single pill with a double-layered structure comprising an inner core of the controlled-release part and an outer layer of the immediate-release part, which encompasses the inner core.

14. The chronotherapeutic combination pharmaceutical formulation of claim 1, which is a capsule comprising a granule of the controlled-release part and a granule of the immediate-release part.

15. The chronotherapeutic combination pharmaceutical formulation of claim 1, which is an uncoated tablet or a coated tablet; optionally wherein
(a) the coated tablet comprises a coating layer of a film former, a film-forming adjuvant or a mixture thereof; or
(b) the coated tablet comprises a coating layer of a film former, a film-forming adjuvant or a mixture thereof, and wherein the coating layer comprises at least one selected from the group consisting of a cellulose derivative, a saccharide derivative, a polyvinyl derivative, a wax, fat, gelatin, polyethylene glycol, ethyl cellulose, titanium oxide, diethyl phthalate and a mixture thereof; or
(c) the coated tablet comprises a coating layer of a film former, a film-forming adjuvant or a mixture thereof, and wherein the coating layer is contained in an amount of 0.5-15 wt% of the total weight of the coated tablet.

16. The chronotherapeutic combination pharmaceutical formulation according to any of claims 1-15, wherein the combination formulation is daily administered at 5-10 p.m.; optionally wherein the statin, a lipid-lowering agent, is released first and the dihydropyridine, a calcium channel blocker, is gradually released 2 hours after the administration, thereby decreasing the interaction between the statin, a lipid-lowering agent, and the dihydropyridine, a calcium channel blocker.

## Patentansprüche

1. Chronotherapeutische, kombinierte, pharmazeutische Formulierung mit kontrollierter Freisetzung, umfassend als Wirkstoffe einen Anteil zur kontrollierten Freisetzung, der ein Dihydropyridin, einen Kalziumkanalblocker, enthält und einen Anteil zur sofortigen Freisetzung, der Statin, ein lipidsenkendes Mittel, enthält.

2. Chronotherapeutische, kombinierte, pharmazeutische Formulierung nach Anspruch 1, wobei die Freisetzungseigenschaft des Kalziumkanalblockers Dihydropyridin und des lipidsenkenden Mittels Statin derart kontrolliert wird, dass der Kalziumkanalblocker Dihydropyridin aufgelöst werden kann, um in der Leber 3 bis 4 Stunden später als das lipidsenkende Mittel Statin absorbiert zu werden.

3. Chronotherapeutische, kombinierte, pharmazeutische Formulierung nach Anspruch 1, wobei die Freisetzungseigenschaften des Kalziumkanalblockers Dihydropyridin und des lipidsenkenden Mittels Statin kontrolliert werden, um die Wechselwirkung zwischen den beiden Arzneimittelbestandteilen, die in der Leber mittels Cytochrom P450 gleichzeitig verstoffwechselt werden, zu reduzieren, so dass der Kalziumkanalblocker Dihydropyridin 2 Stunden nach dem Einsetzen der Freisetzung des lipidsenkenden Mittels Statin freigesetzt werden kann.

4. Chronotherapeutische, kombinierte, pharmazeutische Formulierung nach Anspruch 1, wobei der Kalziumkanalblocker Dihydropyridin ausgewählt ist aus der Gruppe bestehend aus Amlodipin, Lercanidipin, Lacidipin und einem pharmazeutisch verträglichen Salz davon; wobei der Kalziumkanalblocker Dihydropyridin optional
(a) Amlodipin und/oder ein pharmazeutisch verträgliches Salz davon oder ein Isomer davon; oder
(b) Amlodipinmaleat oder Amlodipinbesylat
ist.

5. Chronotherapeutische, kombinierte, pharmazeutische Formulierung nach Anspruch 1, wobei die Formulierung 1 bis 20 mg des Kalziumkanalblockers Dihydropyridin umfasst.

6. Chronotherapeutische, kombinierte, pharmazeutische Formulierung nach Anspruch 1, wobei der Anteil zur kontrollierten Freisetzung ein die Freisetzung kontrollierendes Material umfasst, ausgewählt aus der Gruppe bestehend aus einem enterischen Polymer, einem wasserunlöslichen Polymer, einer hydrophoben Verbindung, einer hydrophilen, nicht-polymeren Verbindung und einem hydrophilen Polymer; wobei optional
(a) das die Freisetzung kontrollierende Material in dem Anteil zur kontrollierten Freisetzung in einer Menge von 10 bis 500 Gewichtsanteilen, bezogen auf 100 Gewichtsanteile des Dihydropyridins, einem Kalziumkanalblocker, enthalten ist;
(b) das enterische Polymer ausgewählt ist aus der Gruppe bestehend aus Polyvinylacetatphthalat, Methacrylsäure-Copolymer, Hydroxypropylmethylcellulosephthalat, Schellack, Celluloseacetatphthalat, Cellulosepropionatphthalat, Eudragit L, Eudragit S und einem Gemisch davon;
(c) das wasserunlösliche Polymer Polyvinylacetat, Methacrylsäure-Copolymer, ausgewählt ist aus der Gruppe bestehend aus Poly(ethylacrylat-co-methylmethacrylat)-Copolymer oder Poly(ethylacrylatmethylmethacrylat-Trimethylaminoethylmethacrylat)-Copolymer, Ethylcellulose, Celluloseacetat und einem Gemisch davon;
(d) die hydrophobe Verbindung ausgewählt ist aus der Gruppe bestehend aus einer Fettsäure und einem Fettsäureester, einem Fettsäurealkohol, einem Wachs, einem anorganischen Material und einem Gemisch davon; oder
(e) jede/jedes aus der hydrophilen, nicht-polymeren Verbindung und dem hydrophilen Polymer ausgewählt ist aus der Gruppe bestehend aus einem Saccharid, einem Cellulosederivat, einem Gummi, einem Protein, einem Polyvinylderivativ, einem Polymethacrylat-Copolymer, einem Polyethylenderivat, einem Carboxyvinylpolymer und einem Gemisch davon.

7. Chronotherapeutische, kombinierte, pharmazeutische Formulierung nach Anspruch 6(d), wobei die Fettsäure und die Fettsäureester ausgewählt sind aus der Gruppe bestehend aus Glycerylpalmitostearat, Glycerylstearat, Glycerylbehenat, Cetylpalmitat, Glycerylmonooleat, Stearinsäure und einem Gemisch davon; der Fettsäurealkohol ausgewählt ist aus der Gruppe bestehend aus Cetostearylalkohol, Cetylalkohol, Stearylalkohol und einem Gemisch davon; das Wachs ausgewählt ist aus der Gruppe bestehend aus Carnaubawachs, Bienenwachs, mikrokristallinem Wachs und einem Gemisch davon; das anorganische Material ausgewählt ist aus der Gruppe bestehend aus Talk, ausgefälltem Calciumcarbonat, zweibasigem Calciumphosphat, Zinkoxid, Titanoxid, Kaolin, Bentonit, Montmorillonit, Veegum und einem Gemisch davon.

8. Chronotherapeutische, kombinierte, pharmazeutische Formulierung nach Anspruch 6(e), wobei das Saccharid ausgewählt ist aus der Gruppe bestehend aus Dextrin, Polydextrin, Dextran, Pektin und Pektinderivat, Alginat, Poly(galacturonsäure), Xylan, Arabinoxylan, Arabinogalactan, Stärke, Hydroxypropylstärke, Amylose, Amylopektin und einem Gemisch davon; wobei
das Cellulosederivat ausgewählt ist aus der Gruppe bestehend aus Hydroxypropylmethylcellulose, Hydroxypropylcellulose, Hydroxymethylcellulose, Hydroxyethylcellulose, Methylcellulose, Carboxymethylcellulose-Natrium, Hydroxypropylmethylcelluloseacetatsuccinat, Hydroxyethylmethylcellulose und einem Gemisch davon;
der Gummi ausgewählt ist aus der Gruppe bestehend aus Guargummi, Johannisbrotgummi, Tragant, Carrageenan, Akaziengummi, Gummi arabicum, Gellangummi, Xanthangummi und einem Gemisch davon;
das Protein ausgewählt ist aus der Gruppe bestehend aus Gelatine, Casein, Zein und einem Gemisch davon;
das Polyvinylderivat ausgewählt ist aus der Gruppe bestehend aus Polyvinylalkohol, Polyvinylpyrrolidon, Poly(vinylacetaldiethylaminoacetat) und einem Gemisch davon;
das Polymethacrylatcopolymer ausgewählt ist aus der Gruppe bestehend aus Poly(butylmethacrylat-(2-dimethylaminoethyl)-Methacrylatmethylmethacrylat)-Copolymer, einem Poly(methacrylsäure-Methylmethacrylat)-Copolymer, einem **Poly**(**methacrylsäure-Ethylacrylat**)-Copolymer und einem Gemisch davon;
das Polyethylenderivat ausgewählt ist aus der Gruppe bestehend aus Polyethylenglycol, Polyethylenoxid und einem Gemisch davon; und
das Carboxyvinylpolymer ein Carbomer ist.

9. Chronotherapeutische, kombinierte, pharmazeutische Formulierung nach Anspruch 1, wobei das Statin, ein lipidsenkendes Mittel, ausgewählt ist aus der Gruppe bestehend aus Simvastatin, Lovastatin, Atorvastatin, Pitavastatin, Rosuvastatin, Fluvastatin, Pravastatin und einem Gemisch davon; und wobei das Statin, ein lipidsenkendes Mittel, optional ausgewählt ist aus der Gruppe bestehend aus Simvastatin, Lovastatin, Atorvastatin und einem Gemisch davon.

10. Chronotherapeutische, kombinierte, pharmazeutische Formulierung nach Anspruch 1, wobei das Statin, ein lipidsenkendes Mittel, in einer Menge von 5 bis 160 mg enthalten ist.

11. Chronotherapeutische, kombinierte, pharmazeutische Formulierung nach Anspruch 1, bei der es sich um eine einzelne Pille mit einer Struktur einer zweiphasigen Matrix handelt, wobei der Anteil zur kontrollierten Freisetzung diskontinuierlich angeordnet ist, wodurch die kontrollierte Freisetzung des Dihydropyridins, einem Kalziumkanalblocker, herbeigeführt wird, und der Anteil zur sofortigen Freisetzung kontinuierlich angeordnet ist, wodurch die sofortige Freisetzung des Statins, einem lipidsenkendem Mittel, hervorgerufen wird.

12. Chronotherapeutische, kombinierte, pharmazeutische Formulierung nach Anspruch 1, wobei der Anteil zur kontrollierten Freisetzung und der Anteil zur sofortigen Freisetzung eine mehrschichtige Struktur bilden.

13. Chronotherapeutische, kombinierte, pharmazeutische Formulierung nach Anspruch 1, bei der es sich um eine einzelne Pille mit einer doppelschichtigen Struktur handelt, die einen inneren Kern aus dem Anteil zur kontrollierten Freisetzung und eine äußere Schicht aus dem Anteil zur sofortigen Freisetzung, die den inneren Kern umschließt, umfasst.

14. Chronotherapeutische, kombinierte, pharmazeutische Formulierung nach Anspruch 1, bei der es sich um eine Kapsel handelt, die ein Korn aus dem Anteil zur kontrollierten Freisetzung und ein Korn aus dem Anteil zur sofortigen Freisetzung umfasst.

15. Chronotherapeutische pharmazeutische Kombinationsformulierung nach Anspruch 1, bei der es sich um eine unbeschichtete Tablette oder eine beschichtete Tablette handelt; wobei optional
(a) die beschichtete Tablette eine Beschichtungsschicht aus einem Filmbildner, einem filmbildenden Adjuvans oder einem Gemisch davon umfasst; oder
(b) die beschichtete Tablette eine Beschichtungsschicht aus einem Filmbildner, einem filmbildenden Adjuvans oder einem Gemisch davon umfasst, und wobei die Beschichtungsschicht mindestens eines aus der Gruppe bestehend aus einem Cellulosederivat, einem Saccharidderivat, einem Polyvinylderivat, einem Wachs, Fett, Gelatine, Polyethylenglykol, Ethylcellulose, Titanoxid, Diethylphthalat und einem Gemisch davon umfasst; oder
(c) die beschichtete Tablette eine Beschichtungsschicht aus einem Filmbildner, einem filmbildenden Adjuvans oder einem Gemisch davon umfasst, und wobei die Beschichtungsschicht in einer Menge von 0,5 bis 15 Gew.-% des Gesamtgewichts der beschichteten Tablette enthalten ist.

16. Chronotherapeutische, kombinierte, pharmazeutische Formulierung nach einem der Ansprüche 1 bis 15, wobei die Kombinationsformulierung täglich zwischen 17 Uhr und 22 Uhr verabreicht wird; wobei optional das Statin, ein lipidsenkendes Mittel, zuerst freigesetzt wird und das Dihydropyridin, ein Kalziumkanalblocker, nach und nach 2 Stunden nach der Verabreichung freigesetzt wird, wodurch die Wechselwirkung zwischen dem Statin, einem lipidsenkenden Mittel, und dem Dihydropyridin, einem Kalziumkanalblocker, reduziert wird.

## Revendications

1. Formulation pharmaceutique combinée chronothérapeutique à libération contrôlée comprenant une partie à libération contrôlée contenant une dihydropyridine, agent bloquant les canaux calciques, et une partie à libération immédiate contenant une statine, agent hypolipémiant, en tant que principes actifs.

2. Formulation pharmaceutique combinée chronothérapeutique selon la revendication 1, dans laquelle la propriété de libération de la dihydropyridine, agent bloquant les canaux calciques, et de la statine, agent hypolipémiant, est contrôlée de telle manière que la dihydropyridine, agent bloquant les canaux calciques, peut être dissoute pour être absorbée dans le foie 3 à 4 heures après la statine, agent hypolipémiant.

3. Formulation pharmaceutique combinée chronothérapeutique selon la revendication 1, dans laquelle les propriétés de libération de la dihydropyridine, agent bloquant les canaux calciques, et de la statine, agent hypolipémiant, sont contrôlées pour réduire l'interaction entre les deux ingrédients médicamenteux métabolisés en même temps dans le foie via le cytochrome P450 de telle sorte que la dihydropyridine, agent bloquant les canaux calciques, peut être libérée deux heures après la libération de la statine, agent hypolipémiant.

4. Formulation pharmaceutique combinée chronothérapeutique selon la revendication 1, dans laquelle la dihydropyridine, agent bloquant les canaux calciques, est sélectionnée dans le groupe consistant en l'amlodipine, la lercanidipine, la lacidipine et un sel pharmaceutiquement acceptable de celles-ci ; facultativement dans laquelle la dihydropyridine, agent bloquant les canaux calciques, est
(a) l'amlodipine et/ou un sel pharmaceutiquement acceptable de celle-ci ou un isomère de celle-ci ; ou
(b) le maléate d'amlodipine ou le bésylate d'amlodipine.

5. Formulation pharmaceutique combinée chronothérapeutique selon la revendication 1, dans laquelle la formulation comprend de 1 à 20 mg de dihydropyridine, agent bloquant les canaux calciques.

6. Formulation pharmaceutique combinée chronothérapeutique selon la revendication 1, dans laquelle la partie à libération contrôlée comprend une substance à libération contrôlée sélectionnée dans le groupe consistant en un polymère entérique, un polymère insoluble dans l'eau, un composé hydrophobe, un composé non polymère hydrophile et un polymère hydrophile ; facultativement dans laquelle
(a) la substance contrôlant la libération dans la partie à libération contrôlée est contenue en une quantité de 10 à 500 parties en poids relativement à 100 parties en poids de la dihydropyridine, agent bloquant les canaux calciques ;
(b) le polymère entérique est sélectionné dans le groupe consistant en le phtalate de polyvinylacétate, un copolymère d'acide méthacrylique, le phtalate d'hydroxypropylméthylcellulose, la gomme laque, l'acétate phtalate de cellulose, le propionate phtalate de cellulose, Eudragit L, Eudragit S et un mélange de ceux-ci ;
(c) le polymère insoluble dans l'eau est l'acétate de polyvinyle, un copolymère d'acide méthacrylique sélectionné dans le groupe consistant en un copolymère de poly(acrylate d'éthyle-co-méthacrylate de méthyle) ou un copolymère de poly(acrylate d'éthyle-méthacrylate de méthyleméthacrylate de triméthyl aminoéthyle), l'éthylcellulose, l'acétate de cellulose et un mélange de ceux-ci ;
(d) le composé hydrophobe est sélectionné dans le groupe consistant en un acide gras et un ester d'acide gras, un alcool d'acide gras, une cire, une substance inorganique et un mélange de ceux-ci ; ou
(e) chacun du composé non polymère hydrophile et du polymère hydrophile est sélectionné dans le groupe consistant en un saccharide, un dérivé de cellulose, une gomme, une protéine, un dérivé de polyvinyle, un copolymère de polyméthacrylate, un dérivé de polyéthylène, un polymère de carboxyvinyle et un mélange de ceux-ci.

7. Formulation pharmaceutique combinée chronothérapeutique selon la revendication 6 (d), dans laquelle l'acide gras et les esters d'acide gras sont sélectionnés dans le groupe consistant en le palmitostéarate de glycéryle, le stéarate de glycéryle, le béhénate de glycéryle, le palmitate de cétyle, le monooléate de glycéryle, l'acide stéarique et un mélange de ceux-ci ; l'alcool d'acide gras est sélectionné dans le groupe consistant en l'alcool cétostéarylique, l'alcool cétylique, l'alcool stéarylique et un mélange de ceux-ci ; la cire est sélectionnée dans le groupe consistant en la cire de carnauba, la cire d'abeille, la cire microcristalline et un mélange de celles-ci ; la substance inorganique est sélectionnée dans le groupe consistant en le talc, le carbonate de calcium précipité, le phosphate de calcium dibasique, l'oxyde de zinc, l'oxyde de titane, le kaolin, la bentonite, la montmorillonite, la Veegum et un mélange de ceux-ci.

8. Formulation pharmaceutique combinée chronothérapeutique selon la revendication 6 (e), dans laquelle le saccharide est sélectionné dans le groupe consistant en la dextrine, la polydextrine, le dextrane, la pectine et un dérivé de la pectine, l'alginate, le poly(acide galacturonique), le xylane, l'arabinoxylane, l'arabinogalactane, l'amidon, l'hydroxypropyl amidon, l'amylose, l'amylopectine et un mélange de ceux-ci ; dans laquelle
le dérivé de cellulose est sélectionné dans le groupe consistant en l'hydroxypropylméthyl cellulose, l'hydroxypropyl cellulose, l'hydroxyméthyl cellulose, l'hydroxyéthyl cellulose, la méthyl cellulose, la carboxyméthyl cellulose calcique, l'acétate succinate d'hydroxypropyl méthyl cellulose, l'hydroxyéthylméthyl cellulose et un mélange de celles-ci ;
la gomme est sélectionnée dans le groupe consistant en la gomme de guar, la gomme de caroubier, la tragacanthe, la carraghénine, la gomme d'acacia, la gomme arabique, la gomme de gellane, la gomme de xanthane et un mélange de celles-ci ;
la protéine est sélectionnée dans le groupe consistant en la gélatine, la caséine, la zéine et un mélange de celles-ci ;
le dérivé de polyvinyle est sélectionné dans le groupe consistant en l'alcool polyvinylique, la polyvinyl pyrrolidone, le poly(diéthylaminoacétate de vinylacétal) et un mélange de ceux-ci ;
le copolymère de polyméthacrylate est sélectionné dans le groupe consistant en un copolymère de poly(méthacrylate de butyle-méthacrylate de (2-diméthylaminoéthyle)-méthacrylate de méthyle), un copolymère de poly(acide méthacrylique-méthacrylate de méthyle), un copolymère de poly(acide méthacrylique-acrylate d'éthyle) et un mélange de ceux-ci ;
le dérivé de polyéthylène est sélectionné dans le groupe consistant en le polyéthylène glycol, l'oxyde de polyéthylène et un mélange de ceux-ci ; et
le polymère de carboxyvinyle est un carbomère.

9. Formulation pharmaceutique combinée chronothérapeutique selon la revendication 1, dans laquelle la statine, agent hypolipémiant, est sélectionnée dans le groupe consistant en la simvastatine, la lovastatine, l'atorvastatine, la pitavastatine, la rosuvastatine, la fluvastatine, la pravastatine et un mélange de celles-ci ; et facultativement dans laquelle la statine, agent hypolipémiant, est sélectionnée dans le groupe consistant en la simvastatine, la lovastatine, l'atorvastatine et un mélange de celles-ci.

10. Formulation pharmaceutique combinée chronothérapeutique selon la revendication 1, dans laquelle la statine, agent hypolipémiant, est contenue en une quantité de 5 à 160 mg.

11. Formulation pharmaceutique combinée chronothérapeutique selon la revendication 1, qui est une pilule unique ayant une structure de matrice à deux phases, dans laquelle la partie à libération contrôlée est placée de manière discontinue, entraînant ainsi la libération contrôlée de la dihydropyridine, agent bloquant les canaux calciques, et la partie à libération immédiate est placée de manière continue, entraînant ainsi la libération immédiate de la statine, agent hypolipémiant.

12. Formulation pharmaceutique combinée chronothérapeutique selon la revendication 1, dans laquelle la partie à libération contrôlée et la partie à libération immédiate forment une structure à couches multiples.

13. Formulation pharmaceutique combinée chronothérapeutique selon la revendication 1, qui est une pilule unique avec une structure double couche comprenant un coeur interne de la partie à libération contrôlée et une couche externe de la partie à libération immédiate, qui englobe le coeur interne.

14. Formulation pharmaceutique combinée chronothérapeutique selon la revendication 1, qui est une capsule comprenant un granule de la partie à libération contrôlée et un granule de la partie à libération immédiate.

15. Formulation pharmaceutique combinée chronothérapeutique selon la revendication 1, qui est un comprimé non pelliculé ou un comprimé pelliculé ; facultativement dans laquelle
(a) le comprimé pelliculé comprend une pellicule d'un agent filmogène, d'un adjuvant filmogène ou d'un mélange de ceux-ci ; ou
(b) le comprimé pelliculé comprend une pellicule d'un agent filmogène, d'un adjuvant filmogène ou d'un mélange de ceux-ci, et dans lequel la pellicule comprend au moins un élément sélectionné dans le groupe consistant en un dérivé de cellulose, un dérivé de saccharide, un dérivé de polyvinyle, une cire, une graisse, la gélatine, le polyéthylèneglycol, l'éthylcellulose, l'oxyde de titane, le phtalate de diéthyle et un mélange de ceux-ci ; ou
(c) le comprimé pelliculé comprend une pellicule d'un agent filmogène, d'un adjuvant filmogène ou d'un mélange de ceux-ci, et dans lequel la pellicule est contenue en une quantité de 0,5 à 15 % en poids du poids total du comprimé pelliculé.

16. Formulation pharmaceutique combinée chronothérapeutique selon l'une quelconque des revendications 1 à 15, dans laquelle la formulation combinée est administrée quotidiennement entre 17 et 22 heures ; facultativement dans laquelle la statine, agent hypolipémiant, est libérée en premier puis la dihydropyridine, agent bloquant les canaux calciques, est progressivement libérée 2 heures après l'administration, réduisant ainsi l'interaction entre la statine, agent hypolipémiant, et la dihydropyridine, agent bloquant les canaux calciques.
